# EUROPEAN PATENT SPECIFICATION

(11) **EP 4 593 942 B1**
(45) Date of publication and mention of the grant of the patent: **20.05.2026**
(21) Application number: 23771960.4
(22) Date of filing: 08.09.2023
(51) Int. Cl.: A61N 1/362, A61N 1/368

(54) **ADAPTIVE CARDIAC CONDUCTION SYSTEM PACING THERAPY FOR MULTI-CHAMBER DEVICES**
ADAPTIVE HERZLEITUNGSSYSTEM-SCHRITTMACHERTHERAPIE FÜR MEHRKAMMERVORRICHTUNGEN
THÉRAPIE DE STIMULATION DE SYSTÈME DE CONDUCTION CARDIAQUE ADAPTATIVE POUR DISPOSITIFS À CHAMBRES MULTIPLES

(30) Priority: 30.09.2022 US 202263411981 P
(43) Date of publication of application: 06.08.2025
(73) Proprietor: Medtronic, Inc., Minneapolis, Minnesota 55432 (US)
(72) Inventor: STADLER, Robert W., Minneapolis, Minnesota 55432 (US)
(74) Representative: Zimmermann & Partner Patentanwälte mbB
(86) International application number: PCT/IB2023/058927
(87) International publication number: WO 2024/069290

(56) References cited:
- EP-A1- 4 023 286
- US-A1- 2006 235 478
- US-A1- 2018 326 215

## Description

The present disclosure relates generally to adaptive cardiac conduction system pacing therapy provided by multi-chamber devices.

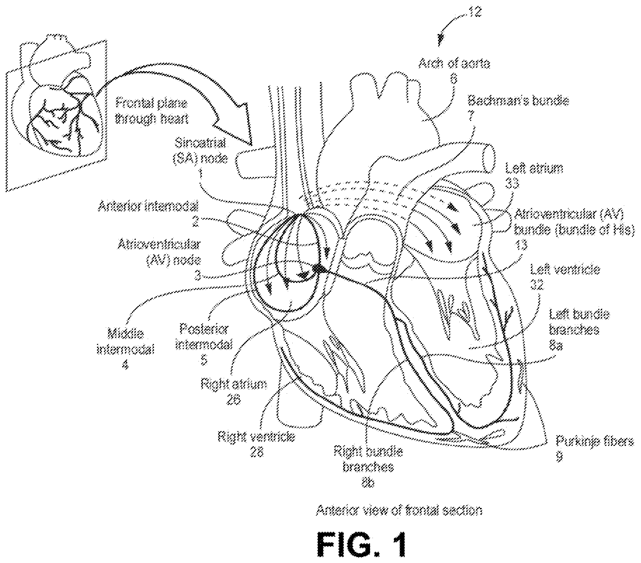

Implantable medical devices (IMDs), such as cardiac pacemakers or implantable cardioverter defibrillators, deliver therapeutic stimulation to patients' hearts thereby improving the lives of millions of patients living with heart conditions. Conventional pacing techniques involve pacing one or more of the four chambers of a patient's heart 12 as illustrated in FIG. 1, including the left atrium 33, the right atrium 26, the left ventricle 32 and the right ventricle 28. One common conventional therapeutic pacing technique that treats a slow heart rate, referred to as bradycardia, involves delivering an electrical pulse to a patient's right ventricular tissue. In response to the electrical pulse, both the right and left ventricles contract. However, the heart beat process may be significantly delayed because the pulse travels from the right ventricle through the left ventricle. The electrical pulse passes through the muscle cells that are referred to as myocytes. Myocyte-to-myocyte conduction may be very slow. Delayed electrical pulses can cause the left ventricle to be unable to maintain synchrony with the right ventricle.

Over time, the left ventricle can become significantly inefficient at pumping blood to the body. In some patients, heart failure can develop such that the heart is too weak to pump blood to the body. Heart failure may be a devastating diagnosis since, for example, fifty percent of the heart failure patients have a life expectancy of five years or less. Another possible cause of heart failure is due to atrial fibrillation, which is an irregular and often very rapid heart rhythm or arrhythmia. During atrial fibrillation, the atria of the heart can beat out of sync with the ventricles of the heart because of the arrythmia of the atria, which can lead to blood clots in the heart and increase the risk of stroke or heart failure, for example.

To avoid potential development of heart failure, some physicians have considered alternative pacing methods that involve the cardiac conduction system. Pacing the cardiac conduction system may quickly conduct electrical pulses (for example, akin to a car driving on a highway), whereas pacing cardiac muscle, or myocardial, tissue may more slowly conduct electrical pulses (for example, akin to a car driving on a dirt road).

The cardiac conduction system includes the sinoatrial node 1, atrial internodal tracts 2, 4, 5 (i.e., anterior internodal 2, middle internodal 4, and posterior internodal 5), atrioventricular node 3, His bundle 13 (also known as the atrioventricular bundle or bundle of His), left bundle branch 8a, and right bundle branch 8b as shown in FIG. 1. The arch of aorta 6 and the Bachman's bundle 7 are also shown in FIG. 1. The sinoatrial node 1, located at the junction of the superior vena cava and right atrium, is considered to be the natural pacemaker of the heart as it continuously and repeatedly emits electrical impulses. The electrical impulses spread through the muscles of right atrium 26 to left atrium 33 to cause synchronous contraction of the atria. The electrical impulses are also carried through atrial internodal tracts to the atrioventricular node 3-the sole connection between the atria and the ventricles. The conduction through the atrioventricular node or atrioventricular nodal tissue takes longer than through the atrial tissue, which results in a delay between the atrial contractions and the start of the ventricular contractions. The atrioventricular delay, which is the delay between atrial contractions and ventricular contractions, allows the atria to empty blood into the ventricles. Then, the valves between the atria and ventricles close in conjunction with ventricular contraction via branches of the bundle of His. The bundle of His, or His bundle, 13 is located in the membranous atrioventricular septum near the annulus of the tricuspid valve. The His bundle 13 splits into the left and right bundle branches 8a, 8b and are formed of specialized fibers called "Purkinje fibers" 9. The Purkinje fibers 9 may be described as being capable of rapidly conducting an action potential down the ventricular septum (VS), spreading the depolarization wavefront quickly through the remaining ventricular myocardium, and producing a coordinated contraction of the ventricular muscle mass.

Traditional cardiac pacing may run the risk of desynchronizing normal ventricular contraction. While cardiac resynchronization therapy (CRT) was invented to correct cardiac dyssynchrony, left ventricular epicardial and right ventricular apical pacing, which is typically used in CRT, may be suboptimal pacing locations. Thus, a ventricular pacing approach that restores normal ventricular synchrony is desirable.

Additionally, patients with long P-wave-to-R-wave intervals (e.g., indicative of first-degree heart block) may suffer from increased risk of atrial fibrillation and heart failure. Additionally, triggered ventricular pacing to correct long P-wave-to-R-wave interval can result in less synchronous ventricular contraction. Thus, pacing that provides both atrioventricular and ventricular synchrony may be desirable that allows for correction of any cardiac conduction abnormalities without introducing dyssynchrony. US 2006/235478 A1 relates to optimization of AV intervals in single ventricle fusion pacing through electrogram morphology.

### SUMMARY

The invention is defined by the appended claims.

This disclosure generally relates to adaptive cardiac conduction system pacing. Further, the illustrative systems, devices, and methods may be described as providing an adaptive cardiac conduction system pacing therapy for dual-chamber and triple-chamber devices to provide atrioventricular and ventricular synchrony despite changing conduction disorders. It is to be understood that dual-chamber devices are capable of delivering pacing therapy to and/or sensing electrical activity of two heart chambers and triple-chamber are capable of delivering pacing therapy to and/or sensing electrical activity of three heart chambers. A multi-chamber device is capable of delivering pacing therapy to and/or sensing electrical activity of two or more heart chambers.

In a least one embodiment, the adaptive pacing may periodically perform a conduction check to determine if the patient has normal P-wave-to-R-wave intervals and normal QRS complex widths. Depending on the patient's conduction at that moment, the pacing mode can switch between a mode that avoids pacing (e.g., inhibited pacing mode), a mode that attempts to fuse conduction system pacing with the patient's remaining intrinsic ventricular activation (e.g., ventricular fusion pacing mode), and a mode that seeks to restore normal atrioventricular synchrony without sacrificing ventricular synchrony (e.g., atrioventricular synchronous pacing mode). If the patient goes into atrial fibrillation, the pacing mode may switch to an atrial fibrillation pacing therapy to produce synchronous contraction slightly faster than native activation. In other words, the illustrative systems, devices, and methods describes adaptive pacing modes for dual-chamber and triple-chamber devices that employ cardiac conduction system pacing to preserve atrioventricular and ventricular synchrony.

Further, patients that receive inadequate resynchronization through cardiac conduction system pacing alone may receive a triple-chamber device including a right atrial lead, cardiac conduction system pacing lead, and a left ventricular coronary sinus lead. These patients will not have normal P-wave-to-R-wave interval and QRS complex width at any time, so the pacing mode that avoids, or inhibits, pacing will be disabled.

Pacing that provides both atrioventricular and ventricular synchrony is highly desirable, allowing for the correction of any cardiac conduction abnormalities without introducing dyssynchrony. Because a patient's conduction disorders can change over time, the illustrative systems, devices, and methods may be described as being responsive thereto with a pacing mode configured for the present conduction disorder.

One illustrative implantable medical device may include a computing apparatus comprising processing circuitry and operably coupled to one or more implantable electrodes comprising a right atrial electrode positionable in the patient's right atrium and a cardiac conduction system pacing electrode positionable proximate a portion of the patient's cardiac conduction system. The computing apparatus may be configured to provide an inhibited pacing mode, a ventricular fusion pacing mode, and an atrioventricular synchronous pacing mode and perform a conduction test. The conduction test may include delaying delivery of cardiac conduction system pacing therapy to allow intrinsic cardiac activation, monitoring intrinsic electrical activity of the patient's heart using the one or more electrodes during intrinsic cardiac activation, determining one or more metrics based on the monitored intrinsic electrical activity, and selecting one of an inhibited pacing mode, a ventricular fusion pacing mode, and an atrioventricular synchronous pacing mode based on the determined one or more metrics. The computing apparatus is further configured to deliver cardiac therapy comprising cardiac conduction system pacing using the cardiac conduction system pacing electrode according to the selected mode.

One illustrative system may include one or more implantable electrodes to sense electrical activity a patient's heart and delivery cardiac therapy to the patient's heart. The one or more implantable electrodes may include a right atrial electrode positionable in the patient's right atrium to one or more of sense electrical activity of and deliver pacing therapy to the right atrium and a cardiac conduction system pacing electrode positionable proximate a portion of the patient's cardiac conduction system to deliver cardiac conduction system pacing therapy to the portion of the patient's cardiac conduction system. The computing apparatus may include processing circuitry and operably coupled to the one or more implantable electrodes. The computing apparatus may be configured to provide an inhibited pacing mode, a ventricular fusion pacing mode, and an atrioventricular synchronous pacing mode and perform a conduction test. A conduction test may include delaying delivery of cardiac conduction system pacing therapy to allow intrinsic cardiac activation, monitoring intrinsic electrical activity of the patient's heart using the one or more electrodes during intrinsic cardiac activation, determining one or more metrics based on the monitored intrinsic electrical activity, and selecting one of an inhibited pacing mode, a ventricular fusion pacing mode, and an atrioventricular synchronous pacing mode based on the determined one or more metrics. The computing apparatus may be further configured to deliver cardiac therapy comprising cardiac conduction system pacing using the cardiac conduction system pacing electrode according to the selected mode.

One illustrative method may include providing an inhibited pacing mode, a ventricular fusion pacing mode, and an atrioventricular synchronous pacing mode and performing a conduction test. The conduction test may include delaying delivery of cardiac conduction system pacing therapy to allow intrinsic cardiac activation and monitoring intrinsic electrical activity of the patient's heart using one or more electrodes during intrinsic cardiac activation. The one or more implantable electrodes may include a right atrial electrode positionable in the patient's right atrium and a cardiac conduction system pacing electrode positionable proximate a portion of the patient's cardiac conduction system. The conduction test may further include determining one or more metrics based on the monitored intrinsic electrical activity and selecting one of an inhibited pacing mode, a ventricular fusion pacing mode, and an atrioventricular synchronous pacing mode based on the determined one or more metrics. The method may further include delivering cardiac therapy comprising cardiac conduction system pacing using the cardiac conduction system pacing electrode according to the selected mode.

The above summary is not intended to describe each embodiment or every implementation of the present disclosure. A more complete understanding will become apparent and appreciated by referring to the following detailed description and claims taken in conjunction with the accompanying drawings.

### BRIEF DESCRIPTION OF THE DRAWINGS

FIG. 1 is a schematic diagram of a heart and conduction system of a patient.
FIG. 2A is a conceptual diagram illustrating an illustrative therapy system that is configured to provide cardiac conduction system pacing therapy to the His bundle using a lead placed in the right atrium.
FIG. 2B is a more detailed conceptual diagram showing the illustrative therapy system of FIG. 2A.
FIG. 2C is a detailed conceptual diagram showing the illustrative therapy system of FIG. 2A but only including two leads.
FIG. 2D is a detailed conceptual diagram showing the illustrative therapy system of FIG. 2A but only including a single lead.
FIG. 3A is a conceptual diagram illustrating an illustrative therapy system that is configured to provide cardiac conduction system pacing therapy to the left bundle branch using a lead placed in the right ventricle.
FIG. 3B is a close-up view of the lead in the patient's heart of FIG. 3A.
FIG. 4A is a conceptual diagram an illustrative therapy system that is configured to provide cardiac conduction system pacing therapy to the left and/or right bundle branches using a lead placed in the right ventricle.
FIG. 4B is a detailed conceptual diagram showing the illustrative therapy system of FIG. 4A but only including two leads.
FIG. 5 is a functional block diagram illustrating an example of a configuration of an implantable medical device of FIGS. 2A-2D.
FIG. 6 is a block diagram of an illustrative method of adaptive cardiac conduction system that may be utilized by the devices of FIGS. 2-5.
FIG. 7A is a state diagram of an illustrative method of selecting a cardiac conduction system pacing mode of FIG. 6 that may be utilized by the dual-chamber devices of FIGS. 2-5.
FIG. 7B is a state diagram of an illustrative method of determining a cardiac conduction system pacing mode of FIG. 6 that may be utilized by the triple-chamber devices of FIGS. 2-5.
FIG. 8 is a block diagram of an illustrative method of selecting an atrial fibrillation pacing mode of FIGS. 6-7 that may be utilized by the devices of FIGS. 2-5.
FIG. 9A is a state diagram of an illustrative method of initially selecting a cardiac conduction system pacing mode of FIG. 6 that may be utilized by the dual-chamber devices of FIGS. 2-5.
FIG. 9B is a state diagram of an illustrative method of switching a cardiac conduction system pacing mode of FIG. 6 that may be utilized by the dual-chamber devices of FIGS. 2-5.

### DETAILED DESCRIPTION

In the following detailed description of illustrative embodiments, reference is made to the accompanying figures of the drawing which form a part hereof, and in which are shown, by way of illustration, specific embodiments which may be practiced. It is to be understood that other embodiments may be utilized, and structural changes may be made without departing from (e.g., still falling within) the scope of the disclosure presented hereby.

Illustrative systems, devices, and methods shall be described with reference to FIGS. 1-9. It will be apparent to one skilled in the art that elements or processes from one embodiment may be used in combination with elements or processes of the other embodiments, and that the possible embodiments of such systems, devices, and methods using combinations of features set forth herein is not limited to the specific embodiments shown in the Figures and/or described herein. Further, it will be recognized that the embodiments described herein may include many elements that are not necessarily shown to scale. Still further, it will be recognized that timing of the processes and the size and shape of various elements herein may be modified but still fall within the scope of the present disclosure, although certain timings, one or more shapes and/or sizes, or types of elements, may be advantageous over others.

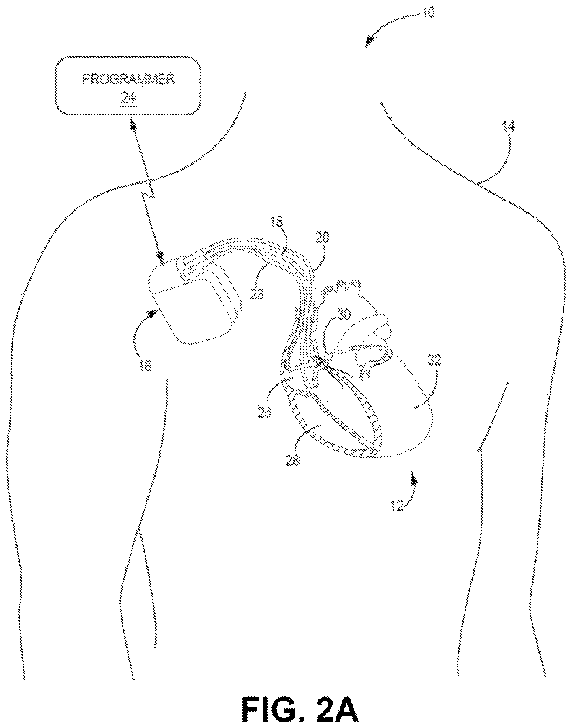

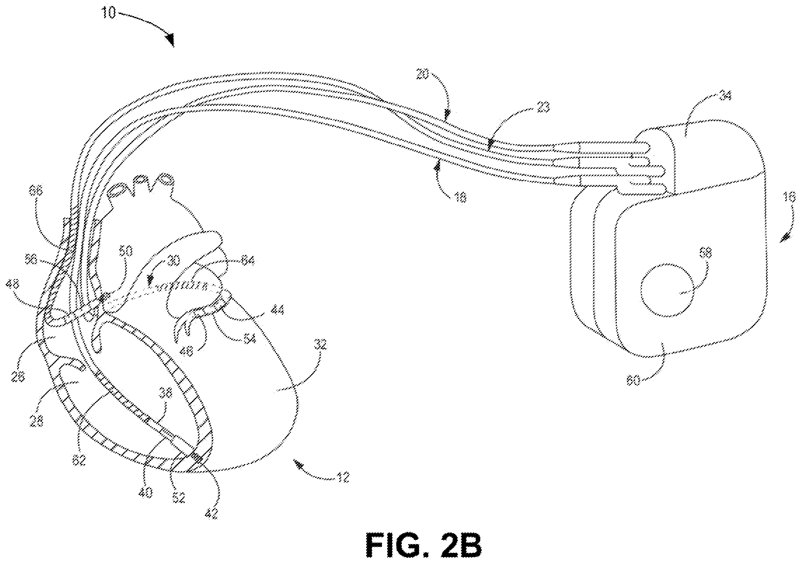

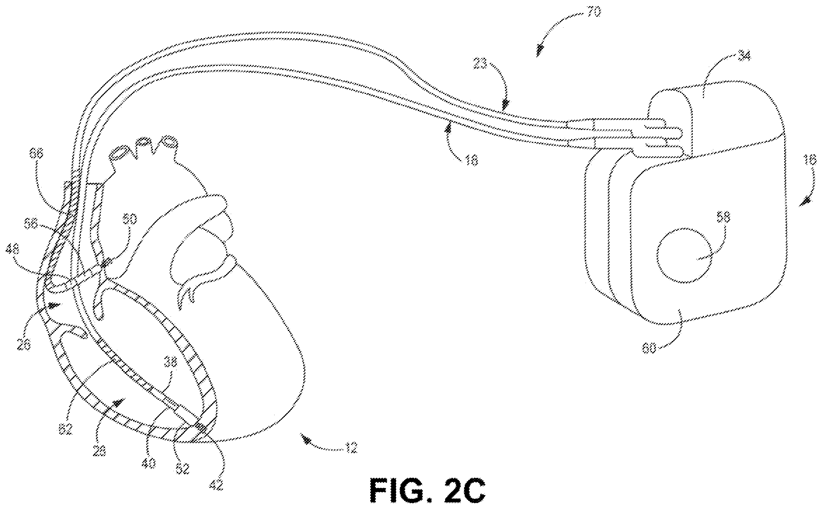

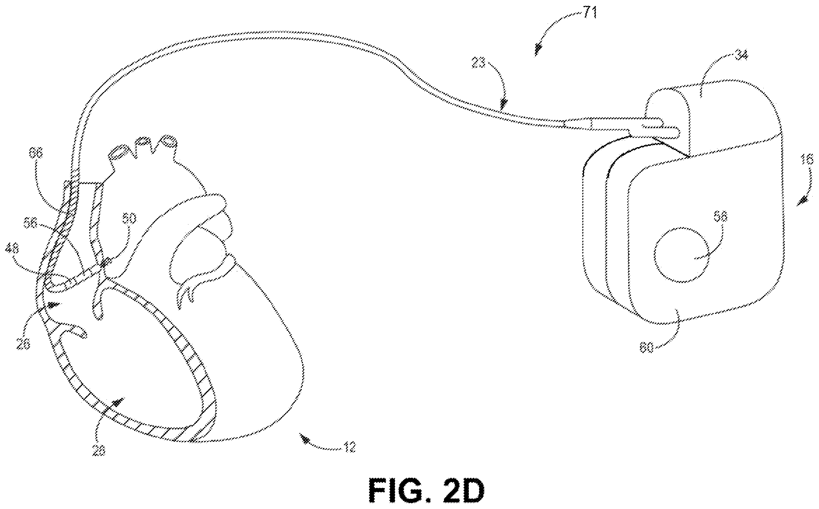

FIG. 1 depicts a schematic diagram of a heart 12 and FIGS. 2-4 depict conceptual diagrams showing illustrative therapy systems that may be used to provide therapy to the heart 12 of a patient 14. The patient 14 ordinarily, but not necessarily, will be a human. As shown in FIGS. 2A-2B, the therapy system 10 may include IMD 16, which is coupled to three leads 18, 20, 23, and a programmer 24. The IMD 16 may be, for example, an implantable pacemaker, cardioverter, and/or defibrillator that provides electrical pulses to the heart 12 via electrodes coupled to one or more of the leads 18, 20, 23. Further non-limiting examples of the IMD 16 include the following: a pacemaker with a medical lead, an implantable cardioverter-defibrillator (ICD), an intracardiac device, a leadless pacing device (LPD), a subcutaneous ICD (S-ICD), and a subcutaneous medical device (e.g., nerve stimulator, inserted monitoring device, etc.).

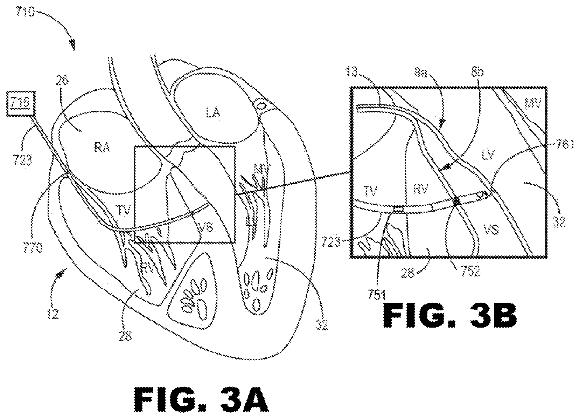

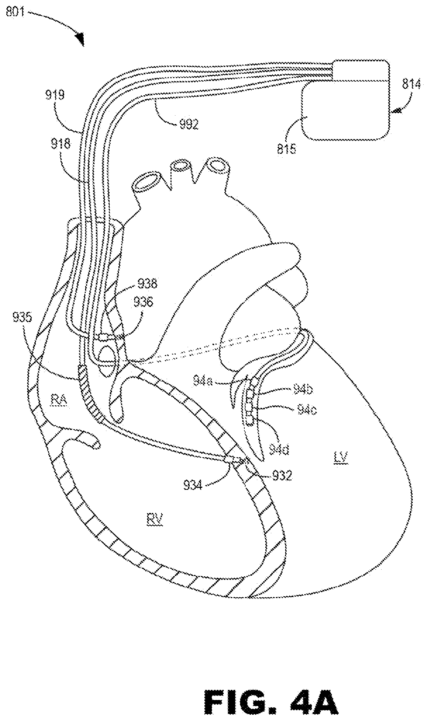

The leads 18, 20, 23 may extend into the heart 12 of the patient 14 to sense electrical activity of the heart 12 and/or deliver electrical stimulation to the heart 12. In the example shown in FIG. 2A, the right ventricular lead 18 extends through one or more veins (not shown), the superior vena cava (not shown), and the right atrium 26, and into the right ventricle 28. The left ventricular coronary sinus lead 20 extends through one or more veins, the vena cava, the right atrium 26, and into the coronary sinus 30 to a region adjacent to the free wall of the left ventricle 32 of the heart 12. The cardiac conduction system pacing therapy lead 23 (e.g., left bundle branch pacing lead, right bundle branch pacing lead, His-bundle pacing lead, etc.) extends through one or more veins and the vena cava, and into the right atrium 26 of heart 12 to pace the cardiac conduction system (e.g., through triangle of Koch region, within the septal wall, proximate and/or in direct contact with the left bundle branch 8a, proximate and/or in direct contact with the right bundle branch 8b, proximate and/or in direct contact with the His bundle 13, etc.). In some embodiments, the cardiac conduction system pacing therapy lead 23 may be positioned within about 1 millimeter of a portion of the cardiac conduction system such as, e.g., the His bundle 13, the left bundle branch 8a, the right bundle branch 8b, etc. In one or more embodiments, a cardiac conduction system therapy lead may be further positioned, or located, through the tricuspid valve into the right ventricle 28 and implanted in the interventricular septum (VS), e.g., about 1 to 2 centimeters in an apical direction as will be described further herein with reference to FIGS. 3A-3B and 4A-4B. One example of a cardiac conduction system pacing therapy lead (e.g., a His lead) can be the SELECTSECURE^{™} 3830. A description of the SELECTSECURE^{™} 3830 is found in the Medtronic model SELECTSECURE^{™} 3830 manual (2013). The SELECTSECURE^{™} 3830 includes two or more conductors with or without lumens.

As used herein, cardiac conduction system pacing therapy refers to any pacing therapy configured to deliver pacing therapy (e.g., pacing pulses, electrical stimulation, etc.) to the cardiac conduction system including, e.g., the His bundle 13, the left bundle branch 8a, the right bundle branch 8b, etc. As used herein, the term "activation" refers to a sensed or paced event. For example, an atrial activation may refer to an atrial sense or event (As) or an atrial pace or artifact of atrial pacing (Ap). As will be described herein, an atrial sense may be detected, or identified, in one or more various signals monitored using one or more various devices or sensors located in one or more various locations. For example, an atrial sense may be detected in a near-field electrical signal from an electrode positioned in the right atrium. Further, for example, an atrial sense may be detected in a far-field electrical signal from an electrode positioned outside of the right atrium such as in the right ventricle or ventricular septum. Still, for example, an atrial sense may be detected in a far-field signal from a mechanical cardiac activation sensor such as an accelerometer or microphone (e.g., a heart sound sensor) positioned in the right atrium or positioned outside of the right atrium such as in the right ventricle or ventricular septum or another portion of the patient's body. Similarly, a ventricular activation may refer to a ventricular sense or event (Vs) or a ventricular pace or artifact of ventricular pacing (Vp), which may be described as ventricular stimulation pulses. In some embodiments, an activation interval can be detected from As or Ap to Vs or Vp, as well as Vp to Vs. In particular, activation intervals may include a pacing (Ap or Vp) to ventricular interval (left ventricular or right ventricular sense) or an atrial-sensing (As) to ventricular-sensing interval (left ventricular or right ventricular).

Illustrative IMDs may be described as delivering one or both of conventional pacing therapy and cardiac conduction system pacing therapy. Conventional, or traditional, pacing therapy may be described as delivering pacing pulses into myocardial tissue that is not part of the cardiac conduction system of the patient's heart such that, e.g., the pacing pulses trigger electrical activation that propagates primarily from one myocardial cell to another myocardial cell (also referred to as "cell-to-cell") as opposed to propagating within the cardiac conduction system prior to the myocardial tissue. For instance, conventional pacing therapy may deliver pacing pulses directly into the muscular heart tissue (e.g., myocardial tissue) that is to be depolarized to provide the contraction of the heart. For example, conventional left ventricular pacing therapy may utilize a left ventricular coronary sinus lead 20 that is implanted so as to extend through one or more veins, the vena cava, the right atrium 26, and into the coronary sinus 30 to a region adjacent to the free wall of the left ventricle 32 of the heart 12 so as to deliver pacing pulses to the myocardial tissue of the free wall of the left ventricle 32.

An illustrative left ventricular lead 20 with a set of spaced apart electrodes is shown in U.S. Pat. Pub. No. WO 2019/104174 A1, filed on May 4, 2012, by Ghosh et al. Illustrative electrodes on leads to form pacing vectors are shown and described in U.S. Pat. No. 8,355,784 B2, and U.S. Pat. No. 8,126,546.

Additionally, the pacing therapy leads 18, 20, 23 may be utilized to deliver left ventricle or left ventricular septal pacing to the ventricular septal wall. At least one of pacing therapy leads 18, 20, 23 may extend through one or more veins, the vena cava, right atrium 26, and into the coronary sinus 30 to a region adjacent to the septal wall of left ventricle 32 of heart 12.

Illustrative cardiac conduction system pacing therapy may be described in, for example, U.S. Pat. App. Pub. No. 2019/0111270 A1 entitled "His Bundle and Bundle Branch Pacing Adjustment" published on April 18, 2019. Illustrative left ventricular septal pacing may be described in, for example, U.S. Pat. App. Ser. No. 16/521,000 entitled "AV Synchronous Septal Pacing" filed on July 24, 2019.

One or more elongated conductors of any of the leads 18, 20, 23 may extend through a hermetic feedthrough assembly, and within an insulative tubular member of the respective lead, and may electrically couple an electrical pulse generator (contained within housing) to one or more electrodes such as, e.g., ring electrodes, tips electrodes, helical electrodes, etc. The conductors may be formed by one or more electrically conductive wires comprising, for example, MP35N alloy known to those skilled in the art, in a coiled or cabled configuration, and the insulative tubular member may be any suitable medical grade polymer, for example, polyurethane, silicone rubber, or a blend thereof. According to one or more illustrative embodiments, the flexible lead body may extend a pre-specified length (e.g., about 10 centimeters (cm) to about 20 cm, or about 15 to 20 cm) from a proximal end to a distal end. The lead body may be less than about 7 French (FR) but typically in the range of about 3 FR to 4 FR in size. In one or more embodiments, about 2 FR size to about 3 FR size lead body is employed.

Cardiac conduction system pacing may include at least one of His bundle pacing and left and/or right bundle branch pacing. Bundle branch pacing may bypass the pathological region and may have a low and stable pacing threshold. In some embodiments, only one of the left bundle branch or the right bundle branch may be paced using one or more pacing leads. In further embodiments, both bundle branches may be paced at the same time (e.g., dual bundle branch pacing), which may mimic intrinsic activation propagation via the His bundle-Purkinje conduction system, e.g., paced activation propagates via both bundle branches to both ventricles for synchronized contraction. His bundle pacing, on the other hand, typically paces the His bundle proximal to the bundle branches. In some embodiments, the IMD 16 may include one, two, or more electrodes located in one or more bundle branches configured for bundle branch pacing.

In some embodiments, the IMD 16 may be an intracardiac pacemaker or leadless pacing device (LPD) configured to pace one or more portions of the cardiac conduction system such as the His bundle. As used herein, "leadless" refers to a device being free of a lead extending out of the heart 12. In other words, a leadless device may have a lead that does not extend from outside of the heart to inside of the heart. Some leadless devices may be introduced through a vein, but once implanted, the leadless devices are free of, or may not include, any transvenous lead and may be configured to provide cardiac therapy without using any transvenous lead. In one or more embodiments, an illustrative LPD for bundle pacing does not use a lead to operably connect to an electrode disposed proximate to the septum when a housing of the device is positioned in the atrium. A leadless electrode may be leadlessly coupled to the housing of the medical device without using a lead between the electrode and the housing.

The IMD 16 may sense electrical signals attendant to the depolarization and repolarization of the heart 12 via various electrodes as shown in FIG. 2B coupled to at least one of leads 18, 20, 23. In some examples, the IMD 16 provides pacing pulses to the heart 12 based on the electrical signals sensed within the heart 12. The configurations of the electrodes used by the IMD 16 for sensing and pacing may be unipolar or bipolar.

The IMD 16 may also provide defibrillation therapy and/or cardioversion therapy via electrodes located on at least one of the leads 18, 20, 23. For example, the IMD 16 may detect atrial arrhythmias of heart 12, such as atrial fibrillation of the atria 26, 33, and then may deliver defibrillation therapy to the heart 12 in the form of electrical pulses. Also, the IMD 16 may detect ventricular arrhythmias of the heart 12, such as ventricular fibrillation of the ventricles 28, 32, and then may deliver defibrillation therapy to the heart 12 in the form of electrical pulses. In some examples, the IMD 16 may be programmed to deliver a progression of therapies, e.g., pulses with increasing energy levels, until fibrillation of the heart 12 is stopped. The IMD 16 may detect fibrillation employing one or more fibrillation detection techniques known in the art.

In some examples, the programmer 24 as shown in FIG. 2A may be a handheld computing device or a computer workstation or a mobile phone. The programmer 24 may include a user interface that receives input from a user. The user interface may include, for example, a keypad and a display, which may for example, be a cathode ray tube (CRT) display, a liquid crystal display (LCD) or light emitting diode (LED) display. The keypad may take the form of an alphanumeric keypad or a reduced set of keys associated with particular functions. The programmer 24 can additionally or alternatively include a peripheral pointing device, such as a mouse, via which a user may interact with the user interface. In some embodiments, a display of the programmer 24 may include a touch screen display, and a user may interact with the programmer 24 via the display. Through the graphical user interface on the programmer 24, a user may configure one or more pacing therapies, select one or more pacing modes, etc.

Additionally, various pacing settings may be adjusted, or configured, based on various sensed signals. For example, various near-field and far-field signals may be sensed by one or more of the electrodes of the IMD 16 and/or other devices operatively coupled thereto. For example, P-wave-to-R-wave interval may be monitored or measured within a near-field or far-field signal and then may be used to adjust, configure, and select cardiac conduction system pacing therapy. Further, for example, QRS width may be monitored or measured within a near-field or far-field signal and then may be used to adjust, configure, and select cardiac conduction system pacing therapy. Still further, for example, one or more of P-wave-to-R-wave interval consistency, T-wave-to-P-wave interval consistency, and P-wave morphology consistency may be monitored or measured within a near-field or far-field signal and then may be used to adjust, configure, and select cardiac conduction system pacing therapy.

The illustrative therapy systems described herein such as IMD 16 may be utilized to deliver cardiac conduction system pacing therapy according to a variety of different modes such as, e.g., inhibited pacing mode, ventricular fusion pacing mode, atrioventricular synchronous pacing mode, atrial fibrillation pacing mode, etc.

The ventricular fusion pacing mode may be configured to deliver cardiac conduction system pacing therapy to provide effective ventricular fusion. Effective ventricular fusion may be described as synchronizing the timing of the left ventricular activation with the activation on the right ventricle. For example, in a fusion pacing configuration, a medical device may deliver one or more pacing pulses in order to pre-excite the left ventricle and synchronize the depolarization of the left ventricle with the depolarization of the earlier contracting right ventricle. The ventricular activation of the left ventricle may "fuse" (or "merge") with the ventricular activation of the right ventricle that is attributable to intrinsic conduction of the heart. In this way, the intrinsic and pacing-induced excitation wave fronts may fuse together such that the depolarization of the left ventricle is resynchronized with the depolarization of the right ventricle.

As used herein, the term "far-field" electrical signal refers to the result of measuring cardiac activity using a sensor, such as an electrode, positioned outside of an area of interest. For example, a far-field electrical signal representing electrical activity of a chamber of interest of the patient's heart may be measured from an electrode positioned in an adjacent chamber (i.e., a chamber different from than that of the chamber of interest that is next to or near the chamber of interest). More specifically, for example, atrial electrical activity, or electrical activity originating one or more both atria, representative of depolarization of the one or both atria may be monitored in a far-field electrical signal measured using an electrode positioned outside of the right atrium such as in the right or left ventricle, or in the ventricular septum. As used herein, the term "near-field" electrical signal refers to the result of measuring cardiac activity using a sensor, such as an electrode, positioned near an area of interest. For example, an electrical signal measured from an electrode positioned on the left side of the patient's ventricular septum is one example of a near-field electrical signal of the patient's LV.

P-wave timing is the time at which a P-wave is detected. Typically, P-wave timing includes using the maximal first derivative of a P-wave upstroke (or the time of the maximal P-wave value). P-wave timing is also used in the device marker channel to indicate the time of the P-wave or the time of atrial activation. P-wave timing may be determined using near-field signals obtained by sensors (e.g., electrodes, accelerometers, heart sound sensors, etc.) positioned in the atria (e.g., the right atrium) and/or far-field near-field signals obtained by sensors (e.g., electrodes, accelerometers, heart sound sensors, etc.) positioned outside of the atria (e.g., the right atrium) such as in the right ventricle and/or ventricular septum.

R-wave timing is the time at which the QRS complex is detected. Typically, R-wave timing includes using the maximal first derivative of an R-wave upstroke (or the time of the maximal R-wave value). R-wave timing is also used in the device marker channel to indicate the time of the R-wave or the time of ventricular activation.

A user, such as a physician, technician, or other clinician, may interact with the programmer 24 to communicate with the IMD 16. For example, the user may interact with the programmer 24 to retrieve physiological or diagnostic information from the IMD 16. Additionally, a user may also interact with the programmer 24 to program the IMD 16, e.g., select values for operational parameters of the IMD 16. The IMD 16 and programmer 24 may communicate via wireless communication using any techniques known in the art. Examples of communication techniques may include, for example, low frequency or radiofrequency (RF) telemetry, but other techniques are also contemplated. In some examples, the programmer 24 may include a programming head that may be placed proximate to the patient's body near the IMD 16 implant site in order to improve the quality or security of communication between the IMD 16 and the programmer 24.

FIG. 2B is a conceptual diagram illustrating the IMD 16 and the leads 18, 20, 23 of the therapy system 10 in greater detail. The triple-chamber IMD 16 may be used for cardiac rhythm therapy and defibrillation or cardioversion therapy (CRT-D). The leads 18, 20, 23 may be electrically coupled to a stimulation generator, a sensing module, or other modules of IMD 16 via connector block 34. In some examples, proximal ends of leads 18, 20, 23 may include electrical contacts that electrically couple to respective electrical contacts within the connector block 34. In addition, in some examples, the leads 18, 20, 23 may be mechanically coupled to the connector block 34 with the aid of set screws, connection pins, or another suitable mechanical coupling mechanism.

Each of the leads 18, 20, 23 includes an elongated, insulative lead body, which may carry any number of concentric coiled conductors separated from one another by tubular, insulative sheaths. In the illustrated example, an optional pressure sensor 38 and bipolar electrodes 40 and 42 are located proximate to a distal end of the right ventricular lead 18. In addition, the bipolar electrodes 44 and 46 are located proximate to a distal end of the left ventricular lead 20 and bipolar electrodes 48 and 50 are located proximate to a distal end of cardiac conduction pacing lead 23. The cardiac conduction system pacing electrode 50 may be used for pacing and/or sensing of the cardiac conduction system tissue (e.g., His bundle or bundle branch tissue).

In FIG. 2B, the pressure sensor 38 is disposed in right ventricle 28 and may respond to an absolute pressure inside right ventricle 28. The pressure sensor 38 may be, for example, a capacitive or piezoelectric absolute pressure sensor. In other examples, the pressure sensor 38 may be positioned within other regions of the heart 12 and may monitor pressure within one or more of the other regions of the heart 12, or the pressure sensor 38 may be positioned elsewhere within or proximate to the cardiovascular system of the patient 14 to monitor cardiovascular pressure associated with mechanical contraction of the heart. Optionally, a pressure sensor in the pulmonary artery can be used that is in communication with the IMD 16.

The electrodes 40, 44 and 48 may take the form of ring electrodes, and the electrodes 42, 46 and 50 may take the form of extendable and/or fixed helix tip electrodes mounted within the insulative electrode heads 52, 54 and 56, respectively. Each of the electrodes 40, 42, 44, 46, 48 and 50 may be electrically coupled to a respective one of the coiled conductors within the lead body of its associated lead 18, 20, 23, and thereby coupled to respective ones of the electrical contacts on the proximal end of the leads 18, 20 23.

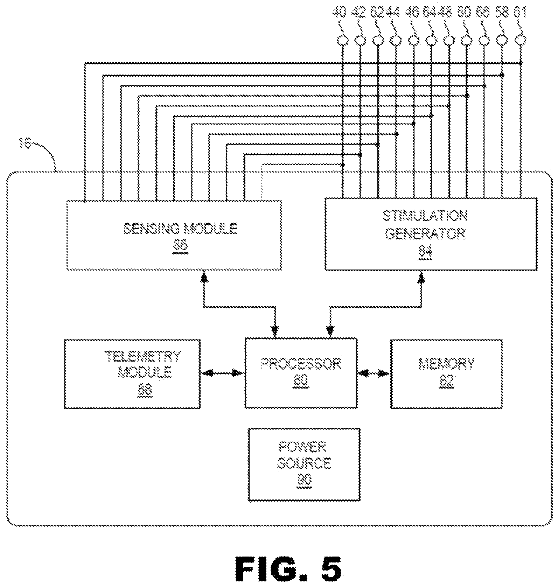

The electrodes 40, 42, 44, 46, 48 and 50 may sense electrical signals attendant to the depolarization and repolarization of the heart 12. The electrical signals are conducted to the IMD 16 via the respective leads 18, 20, 23. In some examples, the IMD 16 also delivers pacing pulses via the electrodes 40, 42, 44, 46, 48, 50 to cause depolarization of cardiac tissue of heart 12. In some examples, as illustrated in FIG. 2B, the IMD 16 may include one or more housing electrodes, such as housing electrode 58, which may be formed integrally with an outer surface of a hermetically sealed housing 60 of the IMD 16 or otherwise coupled to the housing 60. In some examples, the housing electrode 58 may be defined by an uninsulated portion of an outward facing portion of the housing 60 of the IMD 16. Other divisions between insulated and uninsulated portions of housing 60 may be employed to define two or more housing electrodes. In some examples, the housing electrode 58 includes substantially all of the housing 60. Any of the electrodes 40, 42, 44, 46, 48, 50 may be used for unipolar sensing or pacing in combination with the housing electrode 58 or for bipolar sensing with two electrodes in the same pacing lead. In one or more embodiments, the housing 60 may enclose a stimulation generator (see FIG. 5) that generates cardiac pacing pulses and defibrillation or cardioversion shocks, as well as a sensing module for monitoring the patient's heart rhythm.

The leads 18, 20, 23 may also include elongated electrodes 62, 64, 66, respectively, which may take the form of a coil. The IMD 16 may deliver defibrillation shocks to the heart 12 via any combination of the elongated electrodes 62, 64, 66, and the housing electrode 58. The electrodes 58, 62. 64, 66 may also be used to deliver cardioversion pulses to the heart 12. The electrodes 62, 64, 66 may be fabricated from any suitable electrically conductive material, such as, but not limited to, platinum, platinum alloy or other materials known to be usable in implantable defibrillation electrodes.

The pressure sensor 38 may be coupled to one or more coiled conductors within the lead 18. In FIG. 2B, the pressure sensor 38 is located more distally on the lead 18 than elongated electrode 62. In other examples, the pressure sensor 38 may be positioned more proximally than the elongated electrode 62, rather than distal to the electrode 62. Further, the pressure sensor 38 may be coupled to another one of the leads 20, 23 in other examples, or to a lead other than the leads 18, 20, 23 carrying stimulation and sense electrodes. In addition, in some examples, the pressure sensor 38 may be self-contained device that is implanted within the heart 12, such as within the ventricular septum separating the right ventricle 28 from the left ventricle 32, or the atrial septum separating the right atrium 26 from the left atrium 33. In such an example, the pressure sensor 38 may wirelessly communicate with the IMD 16.

FIGS. 2C-2D are conceptual diagrams illustrating additional examples of a dual-chamber therapy system 70 and a single-chamber therapy system 71, respectively. The therapy system 70 is similar to therapy system 10 of FIGS. 2A-2B, but includes two leads 18, 23, rather than three leads. The therapy system 70 may utilize the IMD 16 configured to deliver, or perform, dual-chamber pacing. The leads 18, 23 are implanted within the right ventricle 28 and the right atrium 26 to pace one or more portions of the cardiac conduction system such as the His bundle or one or both bundle branches, respectively. The therapy system 71 is similar to therapy system 10 of FIGS. 2A-2B, but includes a single lead 23, rather than three leads. The therapy system 71 may utilize the IMD 16 configured to deliver, or perform, single-chamber pacing. The lead 23 is implanted the right atrium 26 to pace one or more portions of the cardiac conduction system such as the His bundle or one or both bundle branches, respectively.

The cardiac conduction system pacing lead 23 may be include an electrode 50 in the form of a helix (also referred to as a helical electrode) that may be positioned proximate to, near, adjacent to, or in, area or portions of the cardiac conduction system such as, e.g., ventricular septum, triangle of Koch, the His bundle, left right bundle branch tissues, and/or right bundle branch tissue. The cardiac conduction system pacing lead 23 may be configured as a bipolar lead or as a quadripolar lead that may be used with a pacemaker device, a CRT-P device or a CRT-ICD.

FIGS. 3A-3B show the patient's heart 12 implanted with an implantable medical electrical lead 723 coupled to an IMD 716 to deliver bundle branch pacing according to one example of an IMD system 710. FIG. 3B is a close-up view of lead 723 in the patient's heart 12 of FIG. 3A. In some embodiments, the electrical lead 723 may be the only lead implanted in the heart 12. In other embodiments as discussed herein, there may be multiple leads implanted in the heart 12. The one or more implantable electrodes may include a pacing electrode implantable proximate the cardiac conduction system or may be implantable in the ventricular septum (VS), to deliver cardiac conduction system pacing therapy, for examples.

In one embodiment, the lead 723 may be configured for dual bundle branch pacing, and the lead 723 may be the same as or similar to lead 23 shown in FIGS. 2A-2B) except that the lead 723 is implanted near the bundle branches in the ventricular septum (VS) from the right ventricle 28 instead of, for example, the His bundle 13. As illustrated, the lead 723 is implanted in the septal wall, or ventricular septum, from the right ventricle 28 toward the left ventricle 32. The lead 723 may not pierce through the wall of the left ventricle 32 or extend into the left ventricular chamber. An electrode 752 and a tissue-piercing electrode 761 may be disposed on a distal end portion of the lead 723, which may also be described as a shaft. The electrode 752 and the tissue-piercing electrode 761 may be the same as or similar to electrode and tissue-piercing electrode 50 shown in FIG. 2B except that the electrode 752 is configured as a cathode electrode to sense or pace the right bundle branch and the electrode 761 is configured to sense or pace the left bundle branch, for example, during dual bundle branch pacing. Accordingly, the electrode 752 may be implanted near right bundle branch 8b, and the electrode 761 may be implanted near the left bundle branch 8a. The electrode 761 may be described as a unipolar cathode electrode, which may be implanted on the left side of the patient's ventricular septum. The electrode 752 may be described as a unipolar cathode electrode, which may be implanted on the right side of the patient's ventricular septum.

During dual bundle branch pacing, both the electrode 752 and the electrode 761 may each deliver a cathodal pulse to achieve synchronized activation, or excitation, of the right bundle branch 8b and the left bundle branch 8a, which may result in synchronized activation of the right ventricle 28 and the left ventricle 32. In some embodiments, the pulses may be delivered at the same time to achieve synchrony. In other embodiments, the pulses may be delivered with a delay to achieve synchrony.

Although the lead 723 as shown in configured for dual bundle branch pacing using the electrodes 752, 761, it is to be understood that the lead 723 or leads similar thereto are considered herein that may only include one of the electrode 752 and the electrode 761, and thus, only configured to deliver cardiac conduction system pacing therapy to one of the right bundle branch and the left bundle branch.

Additionally, the lead 723 may include a right atrial electrode 770 disposed more proximal to the electrode 752 and the electrode 761 along the lead 723. The right atrial electrode 770 may be positioned in or near the right atrium 26 and may function as an anode for cathodal pulses from the electrode 752 and/or the electrode 761. Further, the right atrial electrode 770 may provide atrial sensing to, e.g., sense atrial depolarizations or activations, to sense or detect atrial fibrillation, etc. Although the lead 723 as shown includes the right atrial electrode 770, it is to be understood that the lead 723 may not include the right atrial electrode 770, and instead, only include one or both of the electrode 752 and the electrode 761.

Additionally, the device system 710 may include a mechanical cardiac activation sensor 751 coupled to the lead 723 as shown in FIG. 3B. As shown in this embodiment, when the distal end of the lead 723 is implanted through the right ventricle 28 into the ventricular septum, the mechanical cardiac activation sensor 751 may be positioned in the right ventricle 28. The mechanical cardiac activation sensor 751 may be a motion sensor (e.g., an accelerometer) and/or a heart sound sensor (e.g., a microphone) that may be used to determined atrial activation or depolarization (e.g., atrial kick) so as to be used to deliver atrioventricular timed cardiac conduction system pacing therapy. In other words, the device system 710 may be configured to monitor mechanical activity of the patient's heart using the mechanical cardiac activation sensor, determine atrial activation based on the monitored mechanical activity, and deliver cardiac conduction system pacing using the cardiac conduction system pacing electrode based on the determined atrial activation. Additionally, in one or more embodiments, the mechanical cardiac activation sensor 751 may be located in a housing of the IMD 716, which not be located within the heart of the patient. For example, the house of the IMD 716 may be positioned subcutaneously with the body of the patient. Furthermore, if the device system 710 includes a leadless device, the mechanical cardiac activation sensor 751 may be located in a housing of the leadless device implanted in the right ventricle 28.

Furthermore, atrial activations determined using the mechanical cardiac activation sensor 751 may be used in conjunction with atrial activations determined using near-field or far-field electrical activity. In at least one embodiment, the atrial activations determined using the mechanical cardiac activation sensor 751 may be used to confirm atrial activations determined using near-field or far-field electrical activity, or vice versa.

FIG. 4A is a conceptual diagram of an illustrative system 801 including an IMD, or pacemaker, 814 configured as a multi-chamber pacemaker, a right atrial pacing and sensing lead 919, a coronary sinus lead 992, and a cardiac conduction system pacing lead 918 configured for delivering bundle branch pacing. The IMD 814 is shown coupled to the right atrial lead 919 carrying a pacing tip electrode 936 and a proximal ring electrode 938 that may be used for sensing right atrial signals and delivering atrial pacing to the right atrium. The coronary sinus lead 992 may be advanced into the RA, through the coronary sinus ostium and into a cardiac vein of the left ventricle for positioning electrodes 94a, 94b, 94c, 94d (collectively "CS electrodes 94") epicardially along the left ventricular myocardium for sensing electrocardiogram signals and pacing the left ventricular myocardium. The coronary sinus lead 992 is shown as a quadripolar lead carrying four electrodes 94a-94d that may be selected in various bipolar pacing electrode pairs for pacing the left ventricular myocardial tissue and for sensing left ventricular epicardial electrocardiogram signals. One of the CS electrodes 94 may be selected in combination with pacemaker housing 815 or a coil electrode 935 for delivering unipolar left ventricular myocardial pacing and/or sensing unipolar ventricular electrocardiogram signals.

In this example, the IMD 814 may be capable of delivering high voltage cardioversion/defibrillation shock therapies for cardioverting or defibrillating the heart in response to detecting a ventricular tachyarrhythmia. As such, the lead 918 is shown carrying a coil electrode 935 for delivering high voltage shock pulses. One or more coil electrodes may be included along one or more of leads 918, 919 or 992 in various examples. A coil electrode such as coil electrode 935 may be selected in a unipolar pacing electrode vector with any of the lead-based tip or ring electrodes 932, 934, 936, 938 or 94 for sensing unipolar electrocardiogram signals for analysis and determination of ventricular conduction conditions. In some instances, the coil electrode 935 may be used with the housing 815 for sensing a near-field electrocardiogram signal for use in determining atrial depolarizations or activations, etc.

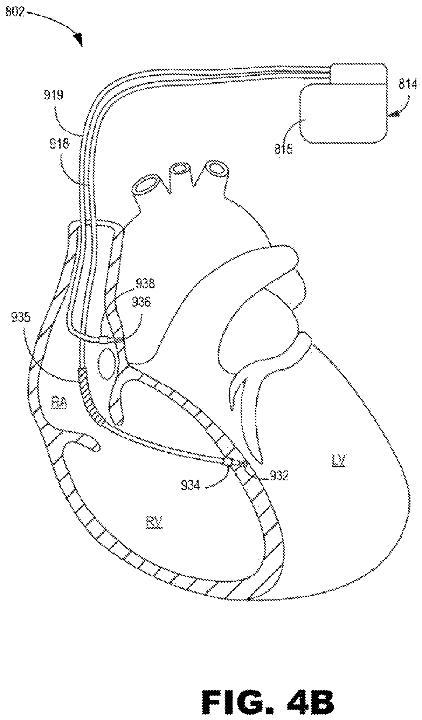

When the pacing lead 918 is positioned for delivering bundle branch pacing, of one or both bundle branches, cardiac conduction system pacing therapy may be combined with traditional ventricular myocardial pacing of the left ventricle using the coronary sinus lead 992 to correct a left ventricular conduction delay and achieve electrical and mechanical synchrony of the left and right ventricles. As such, in some examples, one or more processors, one or more processing circuits, or a computing apparatus of the IMD 814 may select a cardiac conduction system pacing therapy plus traditional left ventricular myocardial pacing therapy that includes, for example, single or bilateral bundle branch pacing, e.g., using the lead 918, combined with left ventricular myocardial pacing using the coronary sinus lead 992. FIG. 4B is a conceptual diagram of another illustrative therapy system 802 that is substantially similar to the illustrative therapy system 801 of FIG. 4A except that the system 802 does not includes coronary sinus lead 992.

The configuration of therapy system 10 illustrated in FIGS. 2-4 are merely examples. In other examples, a therapy system may include epicardial leads and/or patch electrodes instead of or in addition to the transvenous leads 18, 20, 23 illustrated in FIGS. 2-4 or other configurations shown or described herein. Further, the IMDs 16, 716, 814 need not be implanted within patient 14. As such, it is to be understood that the illustrative therapy systems described herein may include any suitable number of leads coupled to IMDs 16, 716, 814, and each of the leads may extend to any location within or proximate to the heart 12. For example, illustrative therapy systems may include three transvenous leads located as illustrated in FIGS. 2A-2C and 4A, a single transvenous lead located as illustrated in FIGS. 3A-3B, or two transvenous leads located as illustrated in FIGS. 2D and 4B.

FIG. 5 is a functional block diagram of one example configuration of the IMD 16. Although the IMD 16 of FIG. 5 is described in terms of the systems shown in FIGS. 2A-2D, it is to be understood that the IMDs 716, 814 may be substantially similar to the IMD 16, and as such, the IMDs 716, 814 may include any or all of the described functionality with respect to the functional block diagram of IMD 16.

The IMD 16 includes a processor 80, a memory 82, a stimulation generator 84 (e.g., electrical pulse generator or signal generating circuit), a sensing module 86 (e.g., sensing circuit), a telemetry module 88, and a power source 90. One or more components of the IMD 16, such as the processor 80, may be contained within a housing of the IMD 16 (e.g., within a housing of a pacemaker). The telemetry module 88, the sensing module 86, or both the telemetry module 88 and the sensing module 86 may be included in a communication interface. The memory 82 includes computer-readable instructions that, when executed by the processor 80, cause the IMD 16 and the processor 80 to perform various functions attributed to the IMD 16 and the processor 80 herein. The memory 82 may include any volatile, non-volatile, magnetic, optical, or electrical media, such as a random-access memory (RAM), read-only memory (ROM), non-volatile RAM (NVRAM), electrically erasable programmable ROM (EEPROM), flash memory, or any other digital media.

The processor 80 may include any one or more of a microprocessor, a controller, a digital signal processor (DSP), an application specific integrated circuit (ASIC), a field-programmable gate array (FPGA), or equivalent discrete or integrated logic circuitry. In some examples, processor 80 may include multiple components, such as any combination of one or more microprocessors, one or more controllers, one or more DSPs, one or more ASICs, or one or more FPGAs, as well as other discrete or integrated logic circuitry. The functions attributed to processor 80 herein may be embodied as software, firmware, hardware or any combination thereof. The processor 80 controls the stimulation generator 84 to select a therapy mode (e.g., select one or more of an inhibited pacing mode, ventricular fusion pacing mode, atrioventricular synchronous pacing mode, atrial fibrillation pacing mode, etc.) and deliver stimulation therapy to the heart 12 according to the selected pacing mode, which may be stored in the memory 82, and various sensing (e.g., atrial depolarizations or activations, ventricular atrial depolarizations or activations, heartrate, P-wave-to-R-wave intervals, etc.). Specifically, the processor 80 may control the stimulation generator 84 to deliver electrical pulses with amplitudes, pulse widths, frequency, or electrode polarities specified by the selected one or more therapy programs and therapy modes.

In some embodiments, the lead 23 may be operably coupled to the electrode 61, which may be used to monitor or pace the right atrium. The stimulation generator 84 may be electrically coupled to the electrodes 40, 42, 44, 46, 48, 50, 58, 61, 62, 64, and 66, e.g., via conductors of the respective lead 18, 20, 23 or, in the case of housing electrode 58, via an electrical conductor disposed within the housing 60 of the IMD 16. The stimulation generator 84 may be configured to generate and deliver electrical stimulation therapy to the heart 12. For example, the stimulation generator 84 may deliver defibrillation shocks to the heart 12 via at least two of the electrodes 58, 62, 64, 66. The stimulation generator 84 may deliver pacing pulses via the ring electrodes 40, 44, 48 coupled to the leads 18, 20, 23, respectively, and/or the helical electrodes 42, 46, 50 of the leads 18, 20, or 23, respectively. The cardiac conduction system pacing therapy can be delivered through the cardiac conduction system lead 23 that is connected to an atrial, right ventricular, or left ventricular connection port of the connector block 34. In some embodiments, the cardiac conduction system pacing therapy can be delivered through the leads 18 and/or 23. In some examples, the stimulation generator 84 delivers pacing, cardioversion, or defibrillation stimulation in the form of electrical pulses. In other examples, the stimulation generator 84 may deliver one or more of these types of stimulation in the form of other signals, such as sine waves, square waves, or other substantially continuous time signals.

The stimulation generator 84 may include a switch module and the processor 80 may use the switch module to select, e.g., via a data/address bus, which of the available electrodes are used to deliver defibrillation shocks or pacing pulses. The switch module may include a switch array, switch matrix, multiplexer, or any other type of switching device suitable to selectively couple stimulation energy to selected electrodes.

The sensing module 86 monitors signals from at least one of the electrodes 40, 42, 44, 46, 48, 50, 58, 61, 62, 64 or 66 in order to monitor electrical activity of the heart 12, e.g., via electrical signals, such as electrocardiogram (ECG) signals and/or electrograms (EGMs). The sensing module 86 may also include a switch module to select which of the available electrodes are used to sense the heart activity. In some examples, the processor 80 may select the electrodes that function as sense electrodes via the switch module within the sensing module 86, e.g., by providing signals via a data/address bus. In some examples, the sensing module 86 includes one or more sensing channels, each of which may include an amplifier. In response to the signals from the processor 80, the switch module may couple the outputs from the selected electrodes to one of the sensing channels.

In some examples, one channel of the sensing module 86 may include an R-wave amplifier that receives signals from the electrodes 44, 46, which are used for pacing and sensing proximate to the left ventricle 32 of the heart 12. Another channel may include another R-wave amplifier that receives signals from the electrodes 40, 42, which are used for pacing and sensing in the right ventricle 28 of the heart 12. In some examples, the R-wave amplifiers may take the form of an automatic gain-controlled amplifier that provides an adjustable sensing threshold as a function of the measured R-wave amplitude of the heart rhythm.

In addition, in some examples, one channel of the sensing module 86 may include a P-wave amplifier that receives signals from electrodes the 48, 50, which are used for pacing and sensing in the right atrium 26 of heart 12. In some examples, the P-wave amplifier may take the form of an automatic gain-controlled amplifier that provides an adjustable sensing threshold as a function of the measured P-wave amplitude of the heart rhythm. Examples of R-wave and P-wave amplifiers are described in U.S. Patent No. 5,117,824 to Keimel et al., which issued on June 2, 1992, and is entitled, "APPARATUS FOR MONITORING ELECTRICAL PHYS SIGNALS". Other amplifiers may also be used. Furthermore, in some examples, one or more of the sensing channels of the sensing module 86 may be selectively coupled to the housing electrode 58, or the elongated electrodes 62, 64, or 66, with or instead of one or more of the electrodes 40, 42, 44, 46, 48 or 50, e.g., for unipolar sensing of R-waves or P-waves in any of the chambers 26, 28, or 32 of the heart 12.

In some examples, the sensing module 86 includes a channel that includes an amplifier with a relatively wider pass band than the R-wave or P-wave amplifiers or a high-resolution amplifier with relatively narrow-pass band for His bundle or bundle branch potential recording. Signals from the selected sensing electrodes that are selected for coupling to this wide-band amplifier may be provided to a multiplexer, and thereafter converted to multi-bit digital signals by an analog-to-digital converter for storage in the memory 82 as an electrogram (EGM). In some examples, the storage of such EGMs in the memory 82 may be under the control of a direct memory access circuit. The processor 80 may employ digital signal analysis techniques to characterize the digitized signals stored in memory 82 to detect and classify the patient's heart rhythm from the electrical signals. The processor 80 may detect and classify the heart rhythm of the patient 14 by employing any of the numerous signal processing methodologies known in the art.

If the IMD 16 is configured to generate and deliver pacing pulses to the heart 12, the processor 80 may include pacer timing and control module, which may be embodied as hardware, firmware, software, or any combination thereof. The pacer timing and control module may include a dedicated hardware circuit, such as an ASIC, separate from other the processor 80 components, such as a microprocessor, or a software module executed by a component of the processor 80, which may be a microprocessor or ASIC. The pacer timing and control module may include programmable counters which control the basic time intervals associated with DDD, VVI, DVI, VDD, AAI, DDI, DDDR, VVIR, DVIR, VDDR, AAIR, DDIR and other modes of single and dual chamber pacing. In the aforementioned pacing modes, "D" may indicate dual chamber, "V" may indicate a ventricle, "I" may indicate inhibited pacing (e.g., no pacing), and "A" may indicate an atrium. The first letter in the pacing mode may indicate the chamber that is paced, the second letter may indicate the chamber in which an electrical signal is sensed, and the third letter may indicate the chamber in which the response to sensing is provided.

Intervals defined by the pacer timing and control module may include atrial and ventricular pacing escape intervals, refractory periods during which sensed P-waves and R-waves are ineffective to restart timing of the escape intervals, and the pulse widths of the pacing pulses. As another example, the pace timing and control module may define a blanking time period and provide signals from sensing module 86 to blank one or more channels, e.g., amplifiers, for a period during and after delivery of electrical stimulation to the heart 12. The durations of these intervals may be determined by the processor 80 in response to stored data in the memory 82. The pacer timing and control module may also determine the amplitude of the cardiac pacing pulses.

During pacing, escape interval counters within the pacer timing/control module may be reset upon sensing of R-waves and P-waves. The stimulation generator 84 may include pacer output circuits that are coupled, e.g., selectively by a switching module, to any combination of the electrodes 40, 42, 44, 46, 48, 50, 58, 61, 62, or 66 appropriate for delivery of a bipolar or unipolar pacing pulse to one of the chambers of the heart 12. The processor 80 may reset the escape interval counters upon the generation of pacing pulses by stimulation generator 84, and thereby control the basic timing of cardiac pacing functions, including anti-tachyarrhythmia pacing.

In some examples, the processor 80 may operate as an interrupt driven device and is responsive to interrupts from pacer timing and control module, where the interrupts may correspond to the occurrences of sensed P-waves and R-waves and the generation of cardiac pacing pulses. Any necessary mathematical calculations to be performed by the processor 80 and any updating of the values or intervals controlled by the pacer timing and control module of the processor 80 may take place following such interrupts. A portion of the memory 82 may be configured as a plurality of recirculating buffers, capable of holding series of measured intervals, which may be analyzed by the processor 80 in response to the occurrence of a pace or sense interrupt to determine whether the patient's heart 12 is presently exhibiting atrial or ventricular tachyarrhythmia.

The telemetry module 88 includes any suitable hardware, firmware, software or any combination thereof for communicating with another device, such as the programmer 24. Under the control of the processor 80, the telemetry module 88 may receive downlink telemetry from and send uplink telemetry to the programmer 24 with the aid of an antenna, which may be internal and/or external. The processor 80 may provide the data to be uplinked to the programmer 24 and the control signals for the telemetry circuit within the telemetry module 88, e.g., via an address/data bus. In some examples, the telemetry module 88 may provide received data to the processor 80 via a multiplexer.

The various components of the IMD 16 are coupled to the power source 90, which may include a rechargeable or non-rechargeable battery. A non-rechargeable battery may be selected to last for several years, while a rechargeable battery may be inductively charged from an external device, e.g., on a daily or weekly basis.

The illustrative systems, devices, and methods described herein may provide adaptive cardiac conduction system pacing therapy that may select an appropriate cardiac conduction system pacing therapy mode based on one or more conditions or parameters measured from the patient.

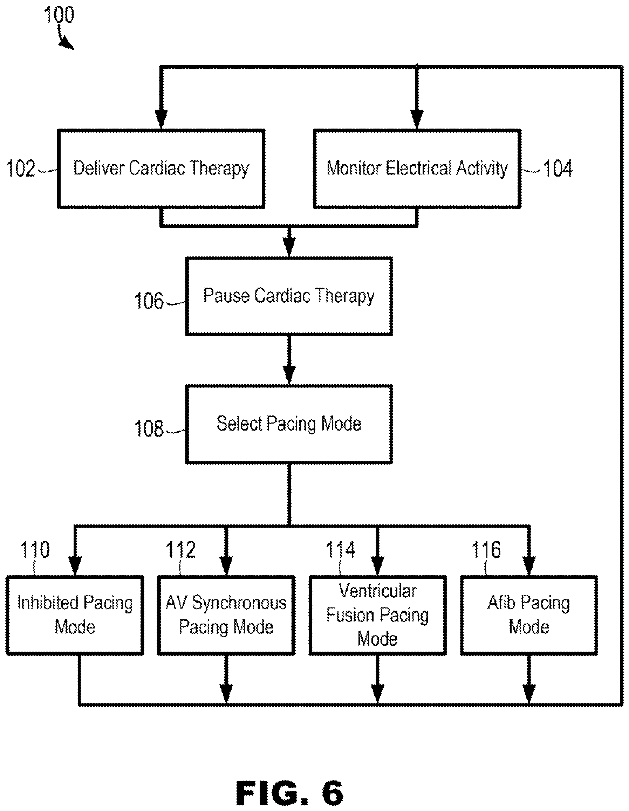

An illustrative method 100 of adaptive cardiac conduction system pacing therapy and delivering such adaptive cardiac conduction system pacing therapy that may be utilized by the devices of FIGS. 2-5 is depicted in FIG. 6. As shown the method 100 may include delivering cardiac therapy 102 and monitoring electrical activity 104. In particular, the method 100 may be performed by an IMD using one or more implantable electrodes to sense electrical activity from a patient's heart 104 and deliver cardiac therapy to the patient's heart 102.

In a single-chamber embodiment, the one or more electrodes may include a cardiac conduction system pacing electrode positionable proximate a portion of the patient's cardiac conduction system to deliver cardiac conduction system pacing therapy to the portion of the patient's cardiac conduction system. The single-chamber embodiment may be configured to deliver four or more different cardiac conduction system pacing therapy modes such as, e.g., an inhibited pacing mode, an atrioventricular synchronous pacing mode, a ventricular fusion pacing mode, and an atrial fibrillation pacing mode, which will be described further herein. Single-chamber adaptive cardiac conduction system pacing therapy may be described in the patent application entitled "Adaptive Cardiac Conduction System Pacing Therapy for Single-Chamber Devices" filed on the same day as the present application, September 30, 2022, corresponding to Medtronic Docket No. A0008129US01.

In a dual-chamber embodiment, the one or more electrodes may include a right atrial electrode positionable in the patient's right atrium to one or more of sense electrical activity of and deliver pacing therapy to the right atrium and a cardiac conduction system pacing electrode positionable proximate a portion of the patient's cardiac conduction system to deliver cardiac conduction system pacing therapy to the portion of the patient's cardiac conduction system. As described herein, a dual-chamber embodiment is configured to deliver pacing therapy to two heart chambers (e.g., right atrium, left ventricle, etc.) and may be located in, or positioned in, one or more chambers. The dual-chamber embodiment may be configured to deliver four or more different cardiac conduction system pacing therapy modes such as, e.g., an inhibited pacing mode, an atrioventricular synchronous pacing mode, a ventricular fusion pacing mode, and an atrial fibrillation pacing mode, which will be described further herein.

In a triple-chamber embodiment, the one or more electrodes may include a right atrial electrode positionable in the patient's right atrium to one or more of sense electrical activity of and deliver pacing therapy to the right atrium, a left ventricular electrode positionable in the coronary sinus to one or more of sense electrical activity of and deliver pacing therapy to the left ventricle, and a cardiac conduction system pacing electrode positionable proximate a portion of the patient's cardiac conduction system to deliver cardiac conduction system pacing therapy to the portion of the patient's cardiac conduction system. As described herein, a triple-chamber embodiment is configured to deliver pacing therapy to three heart chambers (e.g., right atrium, right ventricle, left ventricle, etc.) and may be located in, or positioned in, one or more chambers. The triple-chamber embodiment may be configured to deliver three or more different cardiac conduction system pacing therapy modes such as, e.g., an atrioventricular synchronous pacing mode, a ventricular fusion pacing mode, and an atrial fibrillation pacing mode, which will be described further herein.

The method 100 may include delivering cardiac conduction system pacing therapy 102 according to one of the modes supported by the cardiac therapy provided. During the delivery of the therapy 102, cardiac electrical activity may be monitored 104 using one or more of the electrodes. In particular, one or more both of intrinsic or paced atrial depolarizations or activations and intrinsic or paced ventricular depolarizations or activations may be sensed in the monitored electrical activity 104 so as to be used by the cardiac therapy 102.

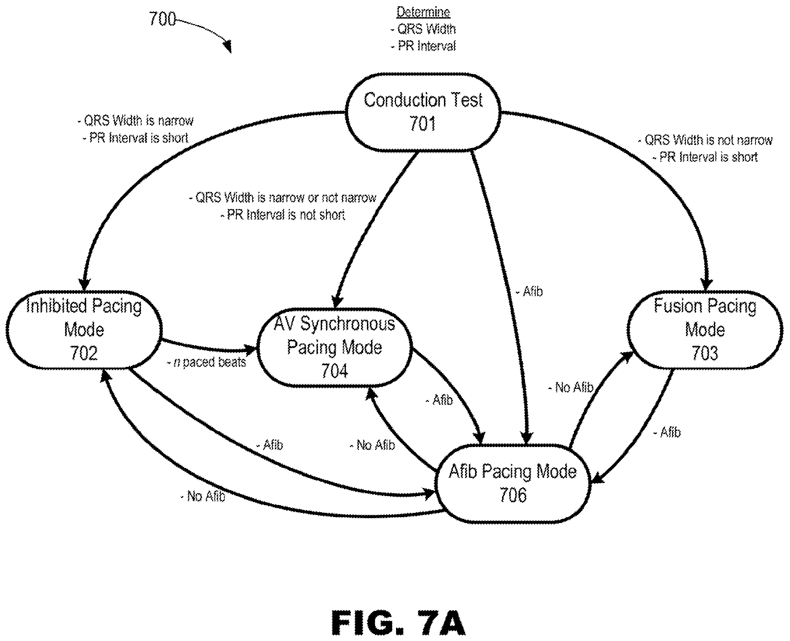

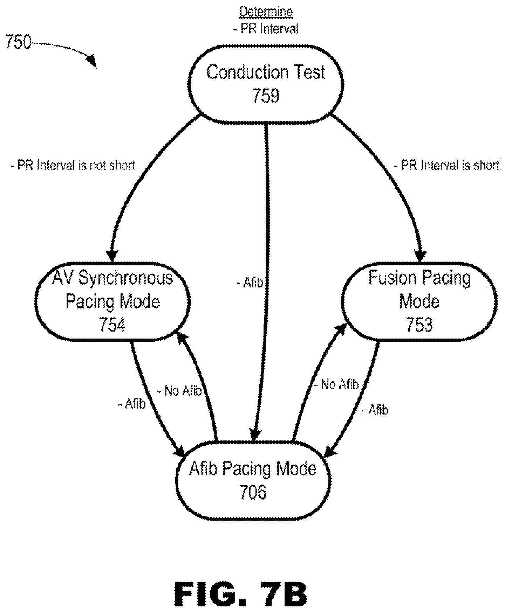

Periodically, the cardiac conduction system pacing therapy being delivered may be paused 106 and a cardiac conduction system pacing therapy pacing mode may be selected 108. In other words, the cardiac conduction system pacing therapy 102 may be temporarily stopped or inhibited 106 such that intrinsic cardiac electrical activity may be monitored for use in selecting the cardiac conduction system pacing therapy mode 108 to be utilized. In at least one embodiment, the atrioventricular delay may be set, or configured to, a time value that is long enough to ensure intrinsic conduction to the ventricles for intrinsic depolarization or activation. In other words, the atrioventricular delay may be extended or lengthened to allow for intrinsic ventricular activation but still deliver ventricular pacing if no intrinsic ventricular activation occurs (e.g., due to AV block, etc.). Selection of the cardiac conduction system pacing therapy 108 for a dual-chamber embodiment is described herein with reference to FIG. 7A. Selection of the cardiac conduction system pacing therapy 108 for a three-chamber embodiment is described herein with reference to FIG. 7B.

The cardiac conduction system pacing therapy may be selected 108 to be one of inhibited pacing mode 110, an atrioventricular synchronous pacing mode 112, a ventricular fusion pacing mode 114, and an atrial fibrillation pacing mode 116. As will be described further herein, three or more different atrial fibrillation pacing modes may be selected or utilized in process 116.

The inhibited pacing mode 110 (also referred to as AAI or AAIR) may be described as delivery of cardiac conduction system pacing when intrinsic ventricular activation does not occur. More specifically, cardiac conduction system pacing may be delivered in response to expiration of an inhibiting atrioventricular delay following an atrial activation without sensing an intrinsic ventricular activation or in response to maintenance of a baseline heart rate during inhibited pacing mode 110. In other words, during an inhibited pacing mode 110, an atrial depolarization or activation sense may trigger, or initiate, the start of an inhibiting atrioventricular delay, which may be selected to allow for an intrinsic ventricular depolarization or activation. If an intrinsic ventricular depolarization or activation occurs prior to the expiration of the inhibiting atrioventricular delay, then cardiac conduction system pacing therapy is withheld, or not delivered, for the cardiac cycle. If an intrinsic ventricular depolarization or activation does not occur prior to the expiration of the inhibiting atrioventricular delay, then cardiac conduction system pacing therapy may be delivered for the cardiac cycle to initiate depolarization or activation of one or both of the ventricles. The inhibiting atrioventricular delay may be between about 200 milliseconds (ms) and about 350 ms, which may depend on whether a sensed inhibiting atrioventricular delay (i.e.. inhibiting atrioventricular delay is triggered, or initiated, in response to sensing of an intrinsic atrial activation or contraction) or a paced inhibiting atrioventricular delay (i.e., inhibiting atrioventricular delay is triggered, or initiated, in response to sensing of pacing the atria) is utilized. A sensed inhibiting atrioventricular delay may be between about 200 ms and about 300 ms, and a paced inhibiting atrioventricular delay may be between about 230 ms and about 350 ms. In at least one embodiment, a sensed inhibiting atrioventricular delay may be 250 ms. In at least one embodiment, a paced inhibiting atrioventricular delay may be 270 ms.

The atrioventricular synchronous pacing mode 112 may be described as delivery of cardiac conduction system pacing in response to expiration of a fixed atrioventricular delay following an atrial activation. In other words, during an atrioventricular synchronous pacing mode 112, an atrial depolarization or activation sense may trigger, or initiate, the start of a fixed atrioventricular delay, which may be selected to allow or provide optimal atrioventricular synchrony. Upon expiration of the fixed atrioventricular delay, the cardiac conduction system pacing therapy may be delivered for the cardiac cycle to initiate depolarization or activation of one or both of the ventricles. The fixed atrioventricular delay may be between about 200 milliseconds (ms) and about 350 ms, which may depend on whether a sensed fixed atrioventricular delay (i.e., fixed atrioventricular delay is triggered, or initiated, in response to sensing of an intrinsic atrial activation or contraction) or a paced fixed atrioventricular delay (i.e., fixed atrioventricular delay is triggered, or initiated, in response to sensing of pacing the atria) is utilized. A sensed fixed atrioventricular delay may be between about 200 ms and about 300 ms, and a paced fixed atrioventricular delay may be between about 230 ms and about 350 ms. In at least one embodiment, a sensed inhibiting atrioventricular delay may be 220 ms. In at least one embodiment, a paced inhibiting atrioventricular delay may be 250 ms.

The ventricular fusion pacing mode 114 may be described as delivery of cardiac conduction system pacing to initiate cardiac depolarization at the same time or substantially the same time as intrinsic ventricular activation. In other words, during a ventricular fusion pacing mode 114, an atrial depolarization or activation sense may trigger, or initiate, the start of a fusion atrioventricular delay, which may be selected to synchronize the cardiac conduction system pacing initiated cardiac depolarization with the intrinsic ventricular activation. Upon expiration of the fusion atrioventricular delay, the cardiac conduction system pacing therapy may be delivered for the cardiac cycle to initiate depolarization or activation of one or both of the ventricles. To achieve ventricular fusion, one or more intrinsic atrioventricular delays may be monitored or measured, and the fusion atrioventricular delay may be determined to be a selected percentage of the shortest monitored intrinsic atrioventricular delay. In particular, when delivering left bundle branch pacing, the monitored intrinsic atrioventricular is measured from an atrial sense or pace to the left bundle branch sense (e.g., the left bundle branch sense may be sensed or monitored using the same electrode to pace the left bundle branch or another electrode located proximate to the left bundle branch). The selected percentage may be between about 50 % and about 90%. In one embodiment, the selected percentage is about 70%.

The atrial fibrillation pacing mode 116 may include an inhibited atrial fibrillation pacing mode, an effective cardiac resynchronization therapy pacing during atrial fibrillation mode, and a conducted atrial fibrillation response (CAFR) mode. The inhibited atrial fibrillation pacing mode (also referred to as DDI or DDIR) may be described as delivery of cardiac conduction system pacing when intrinsic ventricular activation does not occur following the previous ventricular activation. More specifically, cardiac conduction system pacing may be delivered in response to expiration of an inhibiting VV delay following the previous ventricular activation without sensing an intrinsic ventricular activation or in response to maintenance of a baseline heart rate (e.g., such as 50 beats per minute). In other words, during the inhibited atrial fibrillation pacing mode, a ventricular depolarization or activation sense may trigger, or initiate, the start of an inhibiting ventricular delay, which may be selected to allow for an intrinsic ventricular depolarization or activation. If an intrinsic ventricular depolarization or activation occurs prior to the expiration of the inhibiting ventricular delay, then cardiac conduction system pacing therapy is withheld, or not delivered, for the cardiac cycle. If an intrinsic ventricular depolarization or activation does not occur prior to the expiration of the inhibiting ventricular delay, then cardiac conduction system pacing therapy may be delivered for the cardiac cycle to initiate depolarization or activation of one or both of the ventricles.

The effective cardiac resynchronization therapy pacing during atrial fibrillation mode may be generally described as a pacing mode that automatically changes the pacing rate to increase effectiveness of cardiac resynchronization therapy during atrial fibrillation. Generally, if a ventricular paced beat is determined to be ineffective, the pacing rate may be increased, and if a ventricular paced beat is determined to be effective, the pacing rate may be decreased. An illustrative effective cardiac resynchronization therapy pacing mode during atrial fibrillation may be described in U.S. Provisional Pat. App. Ser. No. 63/393,072 filed on July 28, 2022, to Stadler et al. and entitled "Advanced Pacing Delay During Atrial Fibrillation".

The CAFR mode may be generally described as a pacing mode that provides low, medium, and high response values to adjust an amount by which the pacing rate is increased beat by beat. For instance, the ventricular pacing rate may adjust slightly upward or downward for each cardiac cycle. In particular, if the current cardiac cycle ends in a ventricular pace, the pacing rate may be decreased, and if the current cardiac cycle ends in a ventricular sense, the pacing rate may be increased. An illustrative effective cardiac resynchronization therapy pacing mode during atrial fibrillation may be described in U.S. Pat. No. 6,434,424 filed on December 22, 1999, to Igel et al. and entitled "Regularization of Ventricular Rate During Atrial Tachyarrhythmia".

As mentioned, the cardiac conduction system pacing therapy pause 106 and a cardiac conduction system pacing therapy pacing mode selection 108 may occur or be executed periodically. In other words, the method 100 may be performed, or executed, at regular intervals or after expiration of a recurring testing time period. The testing time period may be between about 2 minutes and about 24 hours. Additionally, the testing time period may be different depending on the selected cardiac conduction system pacing therapy pacing mode. For example, if the inhibited pacing mode 110 is selected, then the testing time period may be 10 hours. Further, in at least one embodiment, if the inhibited pacing mode 110 is selected, then QRS complex width may be re-checked in response to high heart rate (e.g., a heart rate above a heart rate threshold). Further, for example, if the atrioventricular synchronous pacing mode 112 is selected, then the testing time period may be initiated as 16 minutes, then decreased to 5 minutes for the second testing time period, then decreased to 4 minutes for the third testing time period, and then decreased to 2 minutes for the fourth and final testing time period. Further, for example, if the ventricular fusion pacing mode 114 is selected, then the testing time period may be initiated as 16 minutes, then decreased to 5 minutes for the second testing time period, then decreased to 4 minutes for the third testing time period, and then decreased to 2 minutes for the fourth and final testing time period. Still further, for example, if the atrial fibrillation pacing mode 116 is selected, then the testing time period may be initiated as 512 minutes, then decreased to 256 minutes for the second testing time period, then decreased to 128 minutes for the third testing time period, and continually decreased by half.

One embodiment of the frequency the cardiac conduction system pacing therapy pause 106 and a cardiac conduction system pacing therapy pacing mode selection 108 is illustrated in Table 1. As shown, Table 1 summarizes the frequency of interval measurements for each of the inhibited pacing mode 110, an atrioventricular synchronous pacing mode 112, a ventricular fusion pacing mode 114, and an atrial fibrillation pacing mode 116. Sometimes, more than one measurement may be used to confirm a change has occurred. For example, the P-wave width and QRS width may be stored in a buffer of the three most recent measurements, and the median of the three most measurements may be the result that is used for all comparisons. Further, the P-wave-to-R-wave interval results may sometimes be used as single measurements and sometimes may be used as a combination of measurements.

**Table 1.**

| | Inhibited pacing mode | Ventricular fusion pacing mode | Atrioventricular synchronous pacing mode | Atrial fibrillation pacing mode |
|---|---|---|---|---|
| **P-wave-to-R-wave Interval** | Every beat | Every minute | Every minute, but progressively less frequent (2, 4, 8 min, ... 16 hours) if P-wave-to-R-wave interval is long. | N/A |
| **P-wave Width** | Every 16 hours | Every 16 hours | Every 16 hours | N/A |
| **QRS Complex Width** | Every minute | Every 4 hours | Every 4 hours | Every minute when QRS complex width is ≤ 110 ms; every 4 hours if QRS width is > 120 ms.* |

| | | | | |
|---|---|---|---|---|
| * The hysteresis stated here applies to transitions from narrow QRS mode to wide QRS mode in the atrial fibrillation pacing mode. If the atrial fibrillation pacing mode is entered from the inhibited pacing mode, the atrial fibrillation pacing mode begins in narrow QRS mode. If the atrial fibrillation pacing mode is entered from the atrioventricular synchronous pacing mode, the atrial fibrillation pacing mode begins in wide QRS mode. If the atrial fibrillation pacing mode is entered from the ventricular fusion pacing mode, ≤110 ms is the threshold for narrow QRS mode in the atrial fibrillation pacing mode. | | | | |

An illustrative method 700 of selecting a cardiac conduction system pacing mode of FIG. 6 when used by an illustrative dual-chamber device is depicted in FIG. 7A. The illustrative dual-chamber device may include a cardiac conduction system pacing electrode positionable proximate a portion of the patient's cardiac conduction system to deliver cardiac conduction system pacing therapy to the portion of the patient's cardiac conduction system. In one embodiment, the cardiac conduction system pacing electrode is positioned proximate the bundle of His and implanted in the right atrium. In another embodiment, the cardiac conduction system pacing electrode is positioned proximate the left bundle branch and implanted in the right ventricle. The dual-chamber device may include one or more leads or be a leadless device. For example, the dual-chamber device may include a first lead extending in the right atrium and a second lead extending in the right ventricle as shown in FIGS. 2C and 4B. Further, for example, the dual-chamber device may include a leadless device implanted in the right ventricle and include a leadlet extending therefrom into the right atrium.

The method 700 may include performing a conduction test 701 during the pause of the delivery cardiac conduction system pacing therapy to determine one or more metrics using the monitored electrical activity. More specifically, in at least one embodiment, the atrioventricular delay may be set, or configured to, a long-time value to ensure intrinsic conduction to the ventricles for intrinsic depolarization or activation. The conduction test 701 may be performed for or over a single cardiac cycle. In other embodiments, the conduction test 701 may be performed for two or more cardiac cycles (e.g., the cardiac conduction system pacing therapy may be paused for two or more cardiac cycles) but still a relatively small number of cardiac cycles such, e.g., as less than 10.

The one or more metrics may include QRS complex width and a P-wave-to-R-wave interval (e.g., paced and/or sensed P-wave-to-R-wave interval). The one or more metrics may then be utilized to determine which cardiac conduction system pacing therapy mode may be selected. For instance, as shown, the inhibited pacing mode 702 may be selected in response to the QRS complex width being narrow and the P-wave-to-R-wave interval being short.

Determination of whether the QRS complex width is narrow may utilize a QRS complex width threshold. For instance, the QRS complex width as determined from the monitored electrical activity during the pause of cardiac conduction system pacing therapy may be compared to the QRS complex width threshold. If the monitored QRS complex width is less than or equal to the QRS complex width threshold, then the monitored QRS complex width may be determined to be narrow. Conversely, if the monitored QRS complex width is greater than the QRS complex width threshold, then the monitored QRS complex width may be determined not to be narrow. The QRS complex width threshold may be between about 110 ms and about 150 ms. In one embodiment, the QRS complex width threshold is 120 ms. Additionally, in one embodiment, when switching from one pacing mode to another. a selected amount of hysteresis may be utilized to, e.g., restrict rapidly switching between pacing modes. For example, the QRS complex width threshold may be 110 ms when initially selecting, or determining, a pacing mode but then may be increase to 120 ms to trigger, or initiate, switching to another pacing mode and/or exiting the current pacing mode.

Determination of whether the P-wave-to-R-wave interval is short may utilize a PR interval threshold. For instance, the P-wave-to-R-wave interval as determined from the monitored electrical activity during the pause of cardiac conduction system pacing therapy may be compared to the PR interval threshold. If the monitored P-wave-toR-wave interval is less than or equal to the PR interval threshold, then the monitored P-wave-to-R-wave interval may be determined to be narrow. Conversely, if the monitored P-wave-to-R-wave interval is greater than the PR interval threshold, then the monitored P-wave-to-R-wave interval may be determined not to be narrow. The PR interval threshold may be between about 180 ms and about 300 ms. In one embodiment, the PR interval threshold is 200 ms when the atria are intrinsically activated, and the PR interval threshold is 250 ms when the atria are paced. Additionally, in one embodiment, when switching from one pacing mode to another, a selected amount of hysteresis may be utilized to, e.g., restrict rapidly switching between pacing modes. For example, if the PR interval threshold may be 200 ms during intrinsic atrial activation when initially selecting, or determining, a pacing mode but then may be increased to 220 ms during intrinsic atrial activation to trigger, or initiate, switching to another pacing mode and/or exiting the current pacing mode. Further, for example, if the PR interval threshold may be 250 ms during paced atrial activation when initially selecting, or determining, a pacing mode but then may be increased to 270 ms during paced atrial activation to trigger, or initiate, switching to another pacing mode and/or exiting the current pacing mode.

Moreover, each of the one or more metrics such as the P-wave-to-R-wave interval and the QRS complex width may be monitored for a selected number of heart beats, or cardiac cycles, and each metric may be evaluated for the selected number of heart beats to further hysteresis. For example, the P-wave-to-R-wave interval may be compared to the PR interval threshold for a selected number, e.g., three, previous heart beats, and a mode switch may not occur unless the P-wave-to-R-wave interval is less than (or equal to or greater than, depending on the mode being switch to) the PR interval threshold for all of the selected number of previous heart beats.

During inhibited pacing mode 702, if cardiac conduction system pacing therapy is delivered for at least a first number, n, of cardiac cycles, or heartbeats, out of a second number of cardiac cycles, or heartbeats, then the atrioventricular synchronous pacing mode 704 may be selected. The second number of cardiac cycles may be larger than the first number of cardiac cycles. In other words, if a selected percentage of previous cardiac cycles were paced, then the atrioventricular synchronous pacing mode 704 may be selected. In at least one embodiment, if two out of the last four cardiac cycles resulted in paced heartbeats, then the atrioventricular synchronous pacing mode 704 may be selected.

Further, as shown, the ventricular fusion pacing mode 703 may be selected in response to the QRS complex width not being narrow and the P-wave-to-R-wave interval being short. Still further, as shown, the atrioventricular synchronous pacing mode 704 may be selected in response to the QRS complex width being narrow or not narrow, and the P-wave-to-R-wave interval not being short. Lastly, if atrial fibrillation is detected following or during the conduction test 701 or during the inhibited pacing mode 702, the ventricular fusion pacing mode 703, or the atrioventricular synchronous pacing mode 704, e.g., using an atrial electrode or another sensor, then the method 700 may select the atrial fibrillation pacing mode 706.

An illustrative method 750 of selecting a cardiac conduction system pacing mode of FIG. 6 when using by an illustrative triple-chamber device is depicted in FIG. 7B. The illustrative three-chamber device may include a cardiac conduction system pacing electrode positionable proximate a portion of the patient's cardiac conduction system to deliver cardiac conduction system pacing therapy to the portion of the patient's cardiac conduction system. In one embodiment, the cardiac conduction system pacing electrode is positioned proximate the bundle of His and implanted in the right atrium. In another embodiment, the cardiac conduction system pacing electrode is positioned proximate the left bundle branch and implanted in the right ventricle. The triple-chamber device may include one or more leads or be a leadless device. For example, the triple-chamber device may include a first lead extending in the right atrium, a second lead extending in the right ventricle, and a third lead extending into the coronary sinus to a region adjacent to the free wall of the left ventricle as shown in FIGS. 2A, 2B, and 4A.

The method 750 may include performing a conduction test 759 during the pause of the delivery cardiac conduction system pacing therapy to determine a P-wave-toR-wave interval using the monitored electrical activity. More specifically, in at least one embodiment, the atrioventricular delay may be set, or configured to, a long-time value to ensure intrinsic conduction to the ventricles for intrinsic depolarization or activation. The conduction test 759 may be performed for or over a single cardiac cycle. In other embodiments, the conduction test 759 may be performed for two or more cardiac cycles (e.g., the cardiac conduction system pacing therapy may be paused for two or more cardiac cycles) but still a relatively small number of cardiac cycles such, e.g., as less than 10.

The P-wave-to-R-wave interval may be utilized to determine which cardiac conduction system pacing therapy mode may be selected. For instance, as shown, the ventricular fusion pacing mode 753 may be selected in response to the P-wave-to-R-wave interval being short and atrioventricular synchronous pacing mode 754 may be selected in response to the P-wave-to-R-wave interval not being short. Lastly, if atrial fibrillation is detected following or during the conduction test 759 or during the ventricular fusion pacing mode 753 or the atrioventricular synchronous pacing mode 754, e.g., using an atrial electrode or another sensor, then the method 750 may select the atrial fibrillation pacing mode 706.

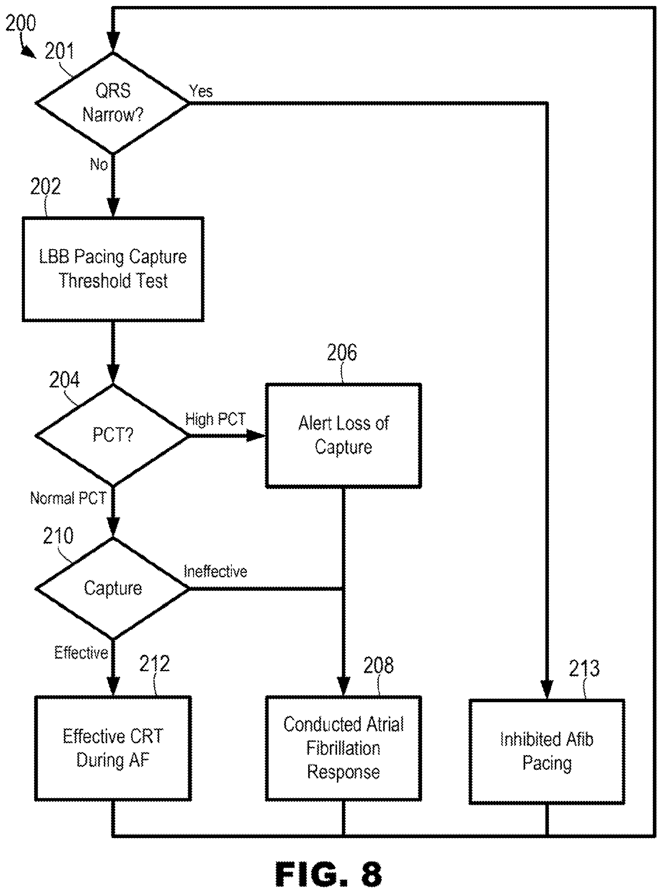

An illustrative method 200 of selecting an atrial fibrillation pacing mode of FIGS. 6-7 that may be utilized by the single-chamber devices of FIGS. 2-5 is depicted in FIG. 8. The method 200 may be executed or performed periodically such, e.g., daily or every 24 hours. At the outset, QRS complex width may be analyzed 201. If the QRS complex width is determined to be narrow 201, then the method 200 may select an inhibited atrial fibrillation pacing mode 213 (also referred to as DDI or DDIR). Further, the method 200 is configured to certify that capture can occur as expected as loss of capture may lead to a "run-away" pacing rate because the intended pacing would always be detected as ineffective. In one or more embodiments, various processes may be used to determine if consistent capture occurs. For example, if at least 4 out of 5 test beats have confirmed capture, then the Effective CRT during AF method may be performed for the next 24 hours.

As shown, if the QRS complex width is determined not to be narrow 201, a pacing capture threshold (PCT) test 202 may be performed or executed. The pacing capture threshold may be compared 204 to determine whether the pacing capture threshold is determined to be normal, indicative of consistent capture, or high, indicative of inconsistent capture. For example, the pacing capture threshold determined during left bundle branch pacing capture threshold (PCT) test 202 may be compared to a normal pacing capture threshold. If the determined pacing capture threshold is greater than the normal pacing capture threshold, then it may be determined that the pacing capture threshold is high indicating inconsistent capture. If the determined pacing capture threshold is less than or equal to the normal pacing capture threshold, then it may be determined that the pacing capture threshold is normal indicating consistent capture. The normal pacing capture threshold may between about 1 Volt (V) and 6 V. In at least one embodiment, the normal pacing capture threshold is 3 V. In at least one embodiment, the normal pacing capture threshold is 2 V.

If a high pacing capture threshold is determined indicating an inability to establish consistent capture, then the method 200 may issue an alert for loss of capture 206 and select the conducted atrial fibrillation response pacing mode 208. If a normal pacing capture threshold is determined indicating an ability to establish consistent capture, then the method 200 may perform a capture determination 210. If capture is determined to be ineffective, then the method 200 may select the conducted atrial fibrillation response pacing mode 208. Conversely, if capture is determined to be effective, then the method 200 may select the effective cardiac resynchronization therapy pacing during atrial fibrillation mode 212.

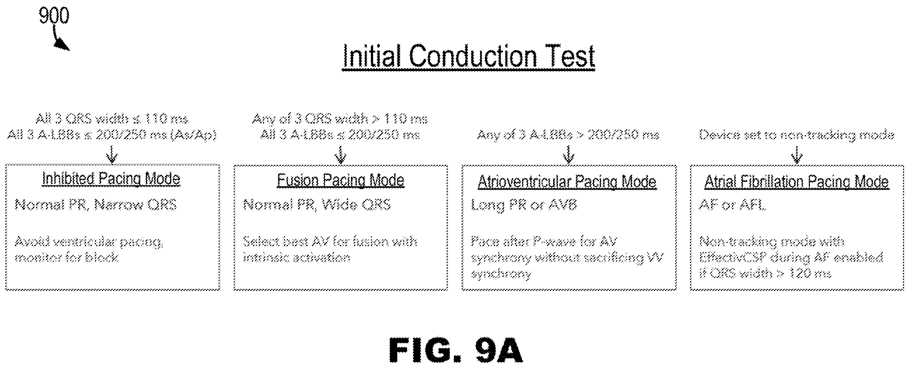

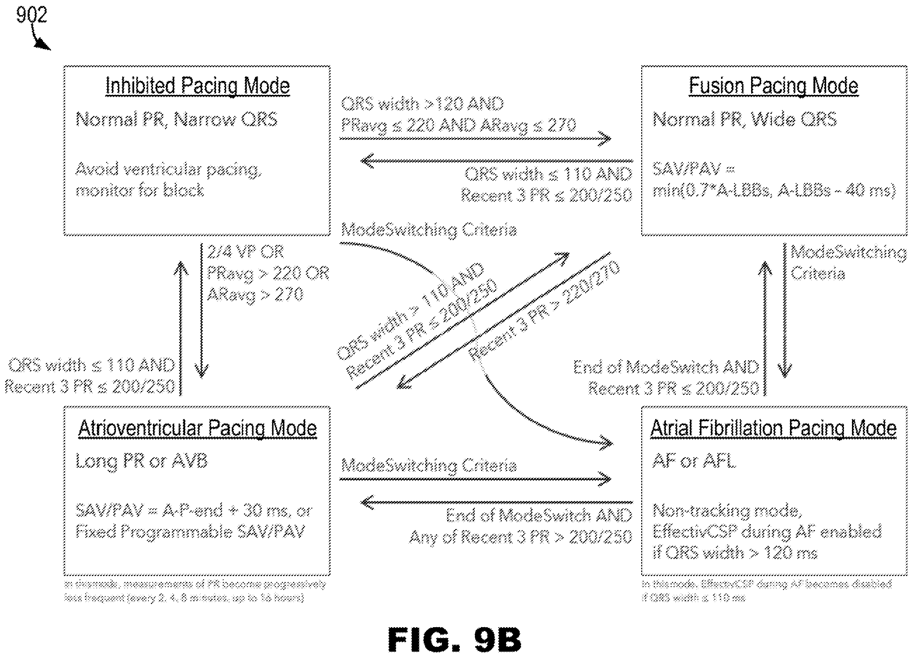

Another illustrative method 900 of initially selecting a cardiac conduction system pacing mode of FIG. 6 that may be utilized by the multi-chamber devices of FIGS. 2-5 is depicted in FIG. 9A, and another illustrative method 902 of switching a cardiac conduction system pacing mode of FIG. 6 that may be utilized by the multi-chamber devices of FIGS. 2-5 is depicted in FIG. 9B. As shown, the QRS width threshold values and PR interval values used by the switching method 902 may be different than some of the QRS width threshold values and PR interval values used by the initial selection method 900, e.g., to provide hysteresis, to avoid rapidly switching between modes, etc.

### ILLUSTRATIVE EXAMPLES

This disclosure has been provided with reference to illustrative embodiments and examples and is not meant to be construed in a limiting sense. As described previously, one skilled in the art will recognize that other various illustrative applications may use the techniques as described herein to take advantage of the beneficial characteristics of the devices and methods described herein. Various modifications of the illustrative embodiments and examples will be apparent upon reference to this description.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

All scientific and technical terms used herein have meanings commonly used in the art unless otherwise specified. The definitions provided herein are to facilitate understanding of certain terms used frequently herein and are not meant to limit the scope of the present disclosure.

Unless otherwise indicated, all numbers expressing feature sizes, amounts, and physical properties used in the specification and claims may be understood as being modified either by the term "exactly" or "about." Accordingly, unless indicated to the contrary, the numerical parameters set forth in the foregoing specification and attached claims are approximations that can vary depending upon the desired properties sought to be obtained by those skilled in the art utilizing the teachings disclosed herein or, for example, within typical ranges of experimental error.

The recitation of numerical ranges by endpoints includes all numbers subsumed within that range (e.g., 1 to 5 includes 1, 1.5, 2, 2.75, 3, 3.80, 4, and 5) and any range within that range. Herein, the terms "up to" or "no greater than" a number (e.g., up to 50) includes the number (e.g., 50), and the term "no less than" a number (e.g., no less than 5) includes the number (e.g., 5).

The terms "coupled" or "connected" refer to elements being attached to each other either directly (in direct contact with each other) or indirectly (having one or more elements between and attaching the two elements). Either term may be modified by "operatively" and "operably," which may be used interchangeably, to describe that the coupling or connection is configured to allow the components to interact to carry out at least some functionality (for example, a mobile user device may be operatively coupled to a cellular network transmit data to or receive data therefrom).

Reference to "one embodiment," "an embodiment," "certain embodiments," or "some embodiments," etc., means that a particular feature, configuration, composition, or characteristic described in connection with the embodiment is included in at least one embodiment of the disclosure. Thus, the appearances of such phrases in various places throughout are not necessarily referring to the same embodiment of the disclosure. Furthermore, the particular features, configurations, compositions, or characteristics may be combined in any suitable manner in one or more embodiments.

As used in this specification the singular forms "a," "an," and "the" encompass embodiments having plural referents, unless the content clearly dictates otherwise. As used in this specification and the appended claims, the term "or" is generally employed in its sense including "and/or" unless the content clearly dictates otherwise.

As used herein, "have," "having," "include," "including," "comprise," "comprising" or the like are used in their open-ended sense, and generally mean "including, but not limited to." It will be understood that "consisting essentially of," "consisting of," and the like are subsumed in "comprising," and the like.

The term "and/or" means one or all of the listed elements or a combination of at least two of the listed elements.

The phrases "at least one of," "comprises at least one of," and "one or more of" followed by a list refers to any one of the items in the list and any combination of two or more items in the list.

## Claims

1. An implantable medical device comprising:
a computing apparatus (50) comprising processing circuitry and operably coupled to one or more implantable electrodes comprising a right atrial electrode (770) positionable in the patient's right atrium and a cardiac conduction system pacing electrode (752, 761) positionable proximate a portion of the patient's cardiac conduction system, wherein the computing apparatus is configured to:
provide an inhibited pacing mode, a ventricular fusion pacing mode, and an atrioventricular synchronous pacing mode;
perform a conduction test comprising:
delaying delivery of cardiac conduction system pacing therapy to allow intrinsic cardiac activation;
monitoring intrinsic electrical activity of the patient's heart using the one or more electrodes during intrinsic cardiac activation;
determining one or more metrics based on the monitored intrinsic electrical activity; and
selecting one of an inhibited pacing mode, a ventricular fusion pacing mode, and an atrioventricular synchronous pacing mode based on the determined one or more metrics; and
deliver cardiac therapy comprising cardiac conduction system pacing using the cardiac conduction system pacing electrode according to the selected mode.

2. The implantable medical device of claim 1, wherein cardiac conduction system pacing comprises:
left bundle branch pacing, wherein the cardiac conduction system pacing electrode is configured to deliver cardiac conduction system pacing therapy to the left bundle branch of the patient's heart, or
His bundle pacing, wherein the cardiac conduction system pacing electrode is configured to deliver cardiac conduction system pacing therapy to the His bundle of the patient's heart.

3. The implantable medical device of claim 1 or 2, wherein the one or more metrics comprises QRS complex width and P-wave-to-R-wave (PR) interval.

4. The implantable medical device of claim 3, wherein the inhibited pacing mode is selected in response to, at least, the QRS complex width being less than or equal to a QRS complex width threshold and the PR interval being less than or equal to a PR interval threshold, wherein the inhibited pacing mode comprises delivery of cardiac conduction system pacing when intrinsic ventricular activation does not occur.

5. The implantable medical device of claim 4, wherein the delivery of cardiac conduction system pacing is in response to expiration of an inhibiting atrioventricular delay following an atrial activation without sensing an intrinsic ventricular activation or in response to maintenance of a baseline heart rate

6. The implantable medical device of claim 4 or 5, wherein the atrioventricular synchronous pacing mode is selected in response to, at least, pacing being delivered for at least two cardiac cycles out of four cardiac cycles.

7. The implantable medical device of any one of claims 4 to 6, wherein the ventricular fusion pacing mode is selected in response to, at least, the QRS complex width being greater than a QRS complex width threshold and the PR interval being less than or equal to a PR interval threshold, wherein the ventricular fusion pacing mode comprises delivery of cardiac conduction system pacing to initiate cardiac depolarization at the same time as intrinsic ventricular activation.

8. The implantable medical device of claim 7, wherein the delivery of cardiac conduction system pacing is in response to expiration of a fusion atrioventricular delay following an atrial activation, wherein the fusion atrioventricular delay is less than a smallest measured intrinsic atrioventricular delay.

9. The implantable medical device of any one of claims 4 to 8, wherein the atrioventricular synchronous pacing mode is selected in response to, at least, the PR interval being greater than a PR interval threshold, wherein the atrioventricular synchronous pacing mode comprises delivery of cardiac conduction system pacing in response to expiration of a fixed atrioventricular delay following an atrial activation.

10. The implantable medical device of any one of claims 1 to 9, wherein the one or more implantable electrodes further comprise a left ventricular electrode (94a, 94b, 94c, 94d) positionable in the coronary sinus to one or more of sense electrical activity of and deliver pacing therapy to the left ventricle, wherein selecting one of an inhibited pacing mode, a ventricular fusion pacing mode, and an atrioventricular synchronous pacing mode based on the determined one or more metrics comprises selecting one of the ventricular fusion pacing mode and the atrioventricular synchronous pacing mode based on a determined PR interval.

11. The implantable medical device of any one of claims 4 to 10, wherein, during delivery of cardiac therapy, an atrial fibrillation pacing mode is selected in response to, at least, atrial fibrillation being sensed using the right atrial electrode.

12. The implantable medical device of claim 11, wherein selection of the atrial fibrillation pacing mode comprises:
selecting an inhibited atrial fibrillation response pacing mode in response to the QRS complex width being less than or equal to a QRS complex width threshold;
selecting a conducted atrial fibrillation response pacing mode in response to a pacing capture threshold (PCT) greater than or equal to a PCT threshold or in response to ineffective capture; and
selecting a cardiac resynchronization pacing mode in response to a pacing capture threshold (PCT) less than the PCT threshold and effective capture.

13. The implantable medical device of any one of claims 1 to 12, wherein the monitoring intrinsic electrical activity of the patient's heart using the one or more electrodes during intrinsic cardiac activation occurs over a testing number of intrinsic heart beats, wherein the testing number of intrinsic heart beats is less than or equal to 10.

14. The implantable medical device of any one of claims 1 to 13, wherein the conduction test is performed periodically in response to expiration of a testing time period, and wherein the computing apparatus adjust the testing time period based on the selected pacing mode.

## Patentansprüche

1. Implantierbare medizinische Vorrichtung, umfassend:
eine Recheneinrichtung (50), umfassend einen Verarbeitungsschaltkreis und die an eine oder mehrere implantierbare Elektroden wirkgekoppelt ist, umfassend eine Elektrode (770) eines rechten Vorhofs, die in dem rechten Vorhof des Patienten positionierbar ist, und eine Stimulationselektrode (752, 761) eines kardialen Erregungsleitungssystems, die nahe einem Abschnitt des kardialen Erregungsleitungssystems des Patienten positionierbar ist, wobei die Recheneinrichtung konfiguriert ist zum:
Bereitstellen eines inhibierten Stimulationsmodus, eines ventrikulären Fusionsstimulationsmodus und eines atrioventrikulären synchronen Stimulationsmodus;
Durchführen eines Leitfähigkeitstests, umfassend:
Verzögern einer Abgabe der Stimulationstherapie des kardialen Erregungsleitungssystems, um eine intrinsische kardiale Aktivierung zu ermöglichen;
Überwachen einer intrinsischen elektrischen Aktivität des Herzens des Patienten unter Verwendung der einen oder der mehreren Elektroden während der intrinsischen kardialen Aktivierung;
Bestimmen einer oder mehrerer Metriken basierend auf der überwachten intrinsischen elektrischen Aktivität; und
Auswählen eines von einem inhibierten Stimulationsmodus, einem ventrikulären Fusionsstimulationsmodus und einem atrioventrikulären synchronen Stimulationsmodus basierend auf der einen oder den mehreren bestimmten Metriken; und
Abgeben einer kardialen Therapie, umfassend eine Stimulation des kardialen Erregungsleitungssystems unter Verwendung der Stimulationselektrode des kardialen Erregungsleitungssystems gemäß dem ausgewählten Modus.

2. Implantierbare medizinische Vorrichtung nach Anspruch 1, wobei die Stimulation des kardialen Erregungsleitungssystems umfasst:
Stimulation eines linken Schenkels, wobei die Stimulationselektrode des kardialen Erregungsleitungssystems konfiguriert ist, um eine Stimulationstherapie des kardialen Erregungsleitungssystems an den linken Schenkel des Herzens des Patienten abzugeben, oder
Stimulation eines His-Bündels, wobei die Stimulationselektrode des kardialen Erregungsleitungssystems konfiguriert ist, um die Stimulationstherapie des kardialen Erregungsleitungssystems an den His-Bündel des Herzens des Patienten abzugeben.

3. Implantierbare medizinische Vorrichtung nach Anspruch 1 oder 2, wobei die eine oder die mehreren Metriken QRS-Komplex-Breite und P-Wellen-zu-R-Wellen-Intervall (PR-Intervall) umfasst.

4. Implantierbare medizinische Vorrichtung nach Anspruch 3, wobei der inhibierte Stimulationsmodus als Reaktion auf, mindestens, die QRS-Komplexbreite, die kleiner als oder gleich einer QRS-Komplexbreitenschwelle ist, und dem PR-Intervall, das kleiner als oder gleich einer PR-Intervallschwelle ist, ausgewählt wird, wobei der inhibierte Stimulationsmodus eine Abgabe der Stimulation des kardialen Erregungsleitungssystems umfasst, wenn keine intrinsische ventrikuläre Aktivierung auftritt.

5. Implantierbare medizinische Vorrichtung nach Anspruch 4, wobei die Abgabe der Stimulation des kardialen Erregungsleitungssystems als Reaktion auf einen Ablauf einer inhibierenden atrioventrikulären Verzögerung nach einer atrialen Aktivierung ohne Erfassen einer intrinsischen ventrikulären Aktivierung oder als Reaktion auf eine Aufrechterhaltung einer Baseline-Herzfrequenz erfolgt.

6. Implantierbare medizinische Vorrichtung nach Anspruch 4 oder 5, wobei der atrioventrikuläre synchrone Stimulationsmodus als Reaktion auf, mindestens, eine Stimulation ausgewählt wird, die für mindestens zwei kardialen Zyklen aus vier kardialen Zyklen abgegeben wird.

7. Implantierbare medizinische Vorrichtung nach einem der Ansprüche 4 bis 6, wobei der ventrikuläre Fusionsstimulationsmodus als Reaktion auf, mindestens, die QRS-Komplexbreite, die größer als eine QRS-Komplexbreitenschwelle ist, und dem PR-Intervall, das kleiner als oder gleich einer PR-Intervallschwelle ist, ausgewählt wird, wobei der ventrikuläre Fusionsstimulationsmodus die Abgabe der Stimulation des kardialen Erregungsleitungssystems umfasst, um eine kardiale Depolarisation zu der gleichen Zeit wie die intrinsische ventrikuläre Aktivierung zu initiieren.

8. Implantierbare medizinische Vorrichtung nach Anspruch 7, wobei die Abgabe der Stimulation des kardialen Erregungsleitungssystems als Reaktion auf den Ablauf einer fusionsatrioventrikulären Verzögerung nach einer atrialen Aktivierung erfolgt, wobei die fusionsatrioventrikuläre Verzögerung kleiner als eine kleinste gemessene intrinsische atrioventrikuläre Verzögerung ist.

9. Implantierbare medizinische Vorrichtung nach einem der Ansprüche 4 bis 8, wobei der atrioventrikuläre synchrone Stimulationsmodus als Reaktion auf, mindestens, das PR-Intervall, das größer als eine PR-Intervallschwelle ist, ausgewählt wird, wobei der atrioventrikuläre synchrone Stimulationsmodus die Abgabe der Stimulation des kardialen Erregungsleitungssystems als Reaktion auf den Ablauf einer fixierten atrioventrikulären Verzögerung nach einer atrialen Aktivierung umfasst.

10. Implantierbare medizinische Vorrichtung nach einem der Ansprüche 1 bis 9, wobei die eine oder die mehreren implantierbaren Elektroden ferner eine linksventrikuläre Elektrode (94a, 94b, 94c, 94d) umfassen, die in dem Koronarsinus positionierbar ist, um eine oder mehrere von Erfassen elektrischer Aktivität des linken Ventrikels und Abgeben einer Stimulationstherapie an diesen, wobei das Auswählen eines von einem inhibierten Stimulationsmodus, einem ventrikulären Fusionsstimulationsmodus und einem atrioventrikulären synchronen Stimulationsmodus basierend auf der einen oder den mehreren bestimmten Metriken das Auswählen eines von dem ventrikulären Fusionsstimulationsmodus und dem atrioventrikulären synchronen Stimulationsmodus basierend auf einem bestimmten PR-Intervall umfasst.

11. Implantierbare medizinische Vorrichtung nach einem der Ansprüche 4 bis 10, wobei während der Abgabe einer kardialen Therapie ein Vorhofflimmernstimulationsmodus als Reaktion auf, mindestens, ein Vorhofflimmern ausgewählt wird, die unter Verwendung der Elektrode des rechten Vorhofs erfasst wird.

12. Implantierbare medizinische Vorrichtung nach Anspruch 11, wobei die Auswahl des Vorhofflimmernstimulationsmodus umfasst:
Auswählen eines inhibierten Vorhofflimmernreaktionstimulationsmodus als Reaktion darauf, dass die QRS-Komplexbreite kleiner als oder gleich einer QRS-Komplexbreitenschwelle ist;
Auswählen eines geleiteten Vorhofflimmernreaktionstimulationsmodus als Reaktion auf eine Stimulationseinfangschwelle (PCT), die größer als oder gleich einer PCT-Schwelle ist, oder als Reaktion auf einen ineffektiven Einfang; und
Auswählen eines kardialen Resynchronisationsstimulationsmodus als Reaktion auf eine Stimulationserfassungsschwelle (PCT), die kleiner als die PCT-Schwelle ist, und eine effektive Erfassung.

13. Implantierbare medizinische Vorrichtung nach einem der Ansprüche 1 bis 12, wobei das Überwachen der intrinsischen elektrischen Aktivität des Herzens des Patienten unter Verwendung der einen oder der mehreren Elektroden während der intrinsischen kardialen Aktivierung über eine Testanzahl von intrinsischen Herzschlägen erfolgt, wobei die Testanzahl von intrinsischen Herzschlägen kleiner als oder gleich 10 ist.

14. Implantierbare medizinische Vorrichtung nach einem der Ansprüche 1 bis 13, wobei der Leitungstest als Reaktion auf den Ablauf einer Testzeitperiode periodisch durchgeführt wird, und wobei die Recheneinrichtung die Testzeitperiode basierend auf dem ausgewählten Stimulationsmodus anpasst.

## Revendications

1. Dispositif médical implantable comprenant :
un appareil informatique (50) comprenant une circuiterie de traitement et couplé de manière opérationnelle à une ou plusieurs électrodes implantables comprenant une électrode auriculaire droite (770) positionnable dans l'oreillette droite du patient et une électrode de stimulation du système de conduction cardiaque (752, 761) positionnable à proximité d'une partie du système de conduction cardiaque du patient, dans lequel l'appareil informatique est configuré pour :
fournir un mode de stimulation inhibée, un mode de stimulation de fusion ventriculaire et un mode de stimulation synchrone auriculoventriculaire ;
effectuer un test de conduction comprenant :
le report d'une administration d'une thérapie de stimulation du système de conduction cardiaque pour permettre une activation cardiaque intrinsèque ;
la surveillance d'une activité électrique intrinsèque du cœur du patient à l'aide de la ou des électrodes pendant l'activation cardiaque intrinsèque ;
la détermination d'une ou plusieurs mesures en fonction de l'activité électrique intrinsèque surveillée ; et
la sélection d'un mode parmi un mode de stimulation inhibée, un mode de stimulation de fusion ventriculaire et un mode de stimulation synchrone auriculoventriculaire en fonction de la ou des métriques déterminées ; et
administrer une thérapie cardiaque comprenant une stimulation du système de conduction cardiaque à l'aide de l'électrode de stimulation du système de conduction cardiaque selon le mode sélectionné.

2. Dispositif médical implantable selon la revendication 1, dans lequel la stimulation du système de conduction cardiaque comprend :
la stimulation de la branche gauche du faisceau, dans lequel l'électrode de stimulation du système de conduction cardiaque est configurée pour administrer une thérapie de stimulation du système de conduction cardiaque à la branche gauche du faisceau du cœur du patient, ou
la stimulation du faisceau de His, dans lequel l'électrode de stimulation du système de conduction cardiaque est configurée pour administrer une thérapie de stimulation du système de conduction cardiaque au faisceau de His du cœur du patient.

3. Dispositif médical implantable selon la revendication 1 ou 2, dans lequel la ou les métriques comprennent la largeur du complexe QRS et l'intervalle entre l'onde P et l'onde R (PR).

4. Dispositif médical implantable selon la revendication 3, dans lequel le mode de stimulation inhibée est sélectionné en réponse, au moins, au fait que la largeur du complexe QRS est inférieure ou égale à un seuil de largeur du complexe QRS et que l'intervalle PR est inférieur ou égal à un seuil d'intervalle PR, dans lequel le mode de stimulation inhibée comprend l'administration d'une stimulation du système de conduction cardiaque lorsque l'activation ventriculaire intrinsèque ne se produit pas.

5. Dispositif médical implantable selon la revendication 4, dans lequel l'administration d'une stimulation du système de conduction cardiaque est en réponse à l'expiration d'un délai auriculoventriculaire inhibiteur suite à une activation auriculaire sans détecter une activation ventriculaire intrinsèque ou en réponse au maintien d'une fréquence cardiaque de base.

6. Dispositif médical implantable selon la revendication 4 ou 5, dans lequel le mode de stimulation synchrone auriculoventriculaire est sélectionné en réponse, au moins, à une stimulation administrée pendant au moins deux cycles cardiaques sur quatre cycles cardiaques.

7. Dispositif médical implantable selon l'une quelconque des revendications 4 à 6, dans lequel le mode de stimulation de fusion ventriculaire est sélectionné en réponse, au moins, au fait que la largeur du complexe QRS est supérieure à un seuil de largeur du complexe QRS et que l'intervalle PR est inférieur ou égal à un seuil d'intervalle PR, dans lequel le mode de stimulation de fusion ventriculaire comprend l'administration d'une stimulation du système de conduction cardiaque pour initier la dépolarisation cardiaque en même temps qu'une activation ventriculaire intrinsèque.

8. Dispositif médical implantable selon la revendication 7, dans lequel l'administration d'une stimulation du système de conduction cardiaque est en réponse à l'expiration d'un délai auriculoventriculaire de fusion suite à une activation auriculaire, dans lequel le délai auriculoventriculaire de fusion est inférieur à un plus petit délai auriculoventriculaire intrinsèque mesuré.

9. Dispositif médical implantable selon l'une quelconque des revendications 4 à 8, dans lequel le mode de stimulation synchrone auriculoventriculaire est sélectionné en réponse, au moins, à l'intervalle PR étant supérieur à un seuil d'intervalle PR, dans lequel le mode de stimulation synchrone auriculoventriculaire comprend l'administration d'une stimulation du système de conduction cardiaque en réponse à l'expiration d'un délai auriculoventriculaire fixe suite à une activation auriculaire.

10. Dispositif médical implantable selon l'une quelconque des revendications 1 à 9, dans lequel la ou les électrodes implantables comprennent en outre une électrode ventriculaire gauche (94a, 94b, 94c, 94d) positionnable dans le sinus coronaire à un ou plusieurs parmi une activité électrique de détection du ventricule gauche et administrer une thérapie de stimulation à celui-ci, dans lequel la sélection d'un mode de stimulation inhibé, un mode de stimulation par fusion ventriculaire et un mode de stimulation synchrone auriculoventriculaire en fonction d'une ou plusieurs mesures déterminées comprennent la sélection d'un mode parmi le mode de stimulation par fusion ventriculaire et le mode de stimulation synchrone auriculoventriculaire en fonction d'un intervalle PR déterminé.

11. Dispositif médical implantable selon l'une quelconque des revendications 4 à 10, dans lequel, pendant l'administration d'une thérapie cardiaque, un mode de stimulation de la fibrillation auriculaire est sélectionné en réponse, au moins, à la détection de la fibrillation auriculaire à l'aide de l'électrode auriculaire droite.

12. Dispositif médical implantable selon la revendication 11, dans lequel la sélection du mode de stimulation de fibrillation auriculaire comprend :
la sélection d'un mode de stimulation de réponse de fibrillation auriculaire inhibée en réponse au fait que la largeur du complexe QRS est inférieure ou égale à un seuil de largeur du complexe QRS ;
la sélection d'un mode de stimulation de réponse de fibrillation auriculaire conduite en réponse à un seuil de capture de stimulation (PCT) supérieur ou égal à un seuil PCT ou en réponse à une capture inefficace ; et
la sélection d'un mode de stimulation par resynchronisation cardiaque en réponse à un seuil de capture de stimulation (PCT) inférieur au seuil PCT et une capture efficace.

13. Dispositif médical implantable selon l'une quelconque des revendications 1 à 12, dans lequel la surveillance de l'activité électrique intrinsèque du cœur du patient à l'aide de la ou des électrodes pendant l'activation cardiaque intrinsèque se produit sur un nombre de tests de battements cardiaques intrinsèques, dans lequel le nombre de tests de battements cardiaques intrinsèques est inférieur ou égal à 10.

14. Dispositif médical implantable selon l'une quelconque des revendications 1 à 13, dans lequel le test de conduction est effectué périodiquement en réponse à l'expiration d'un laps de temps de test, et dans lequel l'appareil informatique ajuste le laps de temps de test en fonction du mode de stimulation sélectionné.
